# EUROPEAN PATENT APPLICATION

(11) **EP 2 633 866 A2**
(43) Date of publication of application: **04.09.2013**
(21) Application number: 13152434.0
(22) Date of filing: 08.10.2004
(51) Int. Cl.: A61K 38/20, A61K 39/395

(54) **Combination therapy**

(30) Priority: 17.10.2003 DK 200301529; 22.10.2003 US 513422 P; 04.05.2004 DK 200400707; 10.05.2004 US 569566 P
(62) Divisional of application: 04762902.7
(71) Applicant: Novo Nordisk A/S, 2880 Bagsværd (DK)
(72) Inventor: Moller, Niels Peter Hundahl, 2100 Kobenhavn (DK); Skak, Kresten, 2860 Soborg (DK); Muller, Jorn Roland, 2830 Virum (DK)
(74) Representative: Griffin, Philippa Jane

(57) **Abstract**

There is provided an antibody and an IL-21 polypeptide for use in treating cancer, wherein said antibody is selected from: (i) HuMax-CD20, alemtuzumab, and Zamyl; (ii) an antibody against tissue factor, laminin-5, EGF receptor, VEGF receptor, PDGF receptor, HER-2/neu, prostate-specific antigen (PSA), prostate stem cell antigen (PSCA), carcinoembryonic antigen (CEA), cancer antigen 125 (CA125), tumor-associated calcium signal transducer antigen (KSA), or VEGF; (iii) an antibody that activates a TRAIL receptor; or (iii) a monoclonal antibody against CTLA-4, leukocyte immunoglobulin-like receptor 1 (LIR), CD94, or NKG2A.

## Description

### FIELD OF THE INVENTION

The present invention relates to IL-21, analogues or derivatives and their use in combination with other pharmaceutical compounds in treatment of cancer and viral infections.

### BACKGROUND OF THE INVENTION

Cytokines generally stimulate proliferation or differentiation of cells of the hematopoietic lineage or participate in the immune and inflammatory response mechanisms of the body. The interleukins are a family of cytokines that mediate immunological responses by producing many cytokines and effect adaptive immunity to antigens. Mature T cells can be activated, i.e., by an antigen or other stimulus, to produce, for example, cytokines, biochemical signaling molecules, or receptors that further influence the fate of the T cell population.

Cytokines produced by the T cell have been classified as type 1 and type 2 (Kelso, A. Immun. Cell Biol. 76:300-317, 1998). Type 1 cytokines include IL-2, IFN-γ, LT-α, and are involved in inflammatory responses, viral immunity, intracellular parasite immunity and allograft rejection. Type 2 cytokines include IL-4, IL-5, IL-6, IL-10 and IL-13, and are involved in humoral responses, helminth immunity and allergic response. Shared cytokines between Type 1 and 2 include IL-3, GM-CSF and TNF-α. There is some evidence to suggest that Type 1 and Type 2 producing T cell populations preferentially migrate into different types of inflamed tissue.

Mature T cells can be activated, i.e., by an antigen or other stimulus, to produce, for example, cytokines, biochemical signaling molecules, or receptors that further influence the fate of the T cell population.

B cells can be activated via receptors on their cell surface including B cell receptor and other accessory molecules to perform accessory cell functions, such as production of cytokines and antibodies.

Natural killer (NK) cells have a common progenitor cell with T cells and B cells, and play a role in immune surveillance. NK cells, which comprise up to 15% of blood lymphocytes, do not express antigen receptors, and therefore do not use MHC recognition as requirement for binding to a target cell. NK cells are involved in the recognition and killing of certain tumor cells and virally infected cells. *In vivo,* NK cells are believed to require activation, however, *in vitro*, NK cells have been shown to kill some types of tumor cells via KIR ligand dependent activation..

The immunology involved in cancer disease includes a variety of different cells derived from the immune system. Compounds which stimulate such responses may be used in combination to provide an improved response to tumor treatment.

IL-21 has been shown to affect a number of different cells of the immune system. Of particular interest is the observed activation of cytotoxic T lymphocytes (CTLs) and NK cells. Both cell types are known to be critically involved in combating tumors. Thus, the presence of intratumoral T lymphocytes is correlated with improved clinical outcome for a number of different cancers (Zhang et al. 2003). However, tumor infiltrating lymphocytes (TILs) are not always sufficiently activated to control tumor growth (Dunn et al. 2002; Kataki et al. 2002; Blohm et al. 2002; Khong and Restifo 2002). Therefore, there is a need for improved therapeutic regimens for treatment, management and prevention of cancers. Another important aspect of IL-21's effect on the immune system is the effect on B cells, which can lead to an improved antibody response against tumor cells and virus-infected cells. The present invention relates to combined use of IL-21 and other modalities for treatment and management of cancer and viral infections.

Although synthetic therapeutic vaccines consisting of one or only few defined antigens have shown some utility in cancer treatment there is a strong need for improvement. The present invention also relates to a timely combination of (i) release of tumor antigens leading to an immune response directed towards these tumor antigens and (ii) the unique ability of IL-21 to induce a sustained cytotoxic T cell (CTL) response. The release of tumor antigens can be obtained with a number of different approaches including the following non-limiting examples: (i) conventional chemotherapy, (ii) induction of apoptosis, (ii) induction of tumor cell death via interference with the blood supply to the tumor, (iv) interference with growth factor stimulation and signal transduction.

### SUMMARY OF THE INVENTION

The invention also provides IL-21, analogues or derivatives thereof in combination with and one or more of the following:
I. Agents that induce tumour cell death or death of virus-infected cells
   a) conventional chemotherapy
   b) radiation therapy
   c) monoclonal antibodies
   d) cell cycle control/apoptosis regulators
   e) growth factor and signal transduction modulators
   f) inhibitors of tumour vascularisation (angiogenesis inhibitors, anti-angiogenesis drugs)
   g) Viral targeting (the use of a recombinant virus to destroy tumour cells)
   h) anti-viral agents
   i) Hormonal agents
II. Agents that enhance the immune response against tumour cells or virus-infected cells
   j) immune system activators
   k) immune system inhibitors (e.g. agents that inhibit immune signals down-regulating the immune response), including anti-anergic agents
   I) therapeutic vaccines
III. Agents that interfere with tumour growth, metastasis or spread of virus-infected cells
   m) integrins, cell adhesion molecules modulators
   n) anti-metastatics
   o) endothelial cell modulators
IV. Internal vaccination.
V. Tissue factor antagonist and other factors influencing the coagulation cascade
   p) anti Factor Xa, anti Factor IIa inhibitors, anti-fibrinogenic agents
   q) pentasaccharides etc.
VI. Immunosuppressive / immunomodulatory agents
   r) agents with influence on T-lymphocyte homing e.g. FTY-720
   s) calcineurin inhibitors
   t) TOR inhibitors

The invention provides a pharmaceutical composition comprising IL-21, an analogue or a derivative thereof, in combination with one or more of the compounds according to the list I-VI and a) - t) above.

The invention provides the use of IL-21, an analogue or a derivative thereof, in combination with and one or more of the compounds selected from the groups I-IV and a) - t) as above for the manufacture of a medicament for the treatment of cancer or viral infections.

The invention provides a method of treating a subject in need thereof with a therapeutically effective amount of a combination of IL-21, an analogue or a derivative thereof together with a one or more of the compounds selected from the groups I-IV and a) - t) as above,

In an embodiment of the invention the diseases to be treated are neoplastic disorders such as metastatic malignant melanoma, renal cell carcinoma, ovarian cancer, small-cell lung cancer, non small-cell lung cancer, breast cancer, colorectal cancer, prostate cancer, pancreatic cancer, bladder cancer, esophageal cancer, cervical cancer, endometrial cancer, lymphoma, and leukaemia.
In more specific aspects of the invention the terms "neoplastic disorders", "cancer" or "tumor growth" are to be understood as referring to all forms of neoplastic cell growth, including both cystic and solid tumors, bone and soft tissue tumors, including both benign and malignant tumors, including tumors in anal tissue, bile duct, bladder, blood cells, bone, bone (secondary), bowel (colon & rectum), brain, brain (secondary), breast, breast (secondary), carcinoid, cervix, children's cancers, eye, gullet (oesophagus), head & neck, kaposi's sarcoma, kidney, larynx, leukaemia (acute lymphoblastic), leukaemia (acute myeloid), leukaemia (chronic lymphocytic), leukaemia (chronic myeloid), leukaemia (other), liver, liver (secondary), lung, lung (secondary), lymph nodes (secondary), lymphoma (hodgkin's), lymphoma (non-hodgkin's), melanoma, mesothelioma, myeloma, ovary, pancreas, penis, prostate, skin, soft tissue sarcomas, stomach, testes, thyroid, unknown primary tumor, vagina, vulva, womb (uterus).

Soft tissue tumors include Benign schwannoma Monosomy, Desmoid tumor, Lipoblastoma, Lipoma, Uterine leiomyoma, Clear cell sarcoma, Dermatofibrosarcoma, Ewing sarcoma, Extraskeletal myxoid chondrosarcoma, Liposarcoma myxoid, Liposarcoma, well differentiated, Alveolar rhabdomyosarcoma, and Synovial sarcoma.

Specific bone tumor include Nonossifying Fibroma, Unicameral bone cyst, Enchondroma, Aneurysmal bone cyst, Osteoblastoma, Chondroblastoma, Chondromyxofibroma, Ossifying fibroma and Adamantinoma, Giant cell tumor, Fibrous dysplasia, Ewing's Sarcoma, Eosinophilic Granuloma, Osteosarcoma, Chondroma, Chondrosarcoma, Malignant Fibrous Histiocytoma, and Metastatic Carcinoma.

Leukaemias referes to cancers of the white blood cells which are produced by the bone marrow. This includes but are not limited to the four main types of leukaemia; acute lymphoblastic (ALL), acute myeloblastic (AML), chronic lymphocytic (CLL) and chronic myeloid (CML).

In another aspect of the invention the diseases to be treated are viral infections such as hepatitis B Virus, Hepatitis C virus, Human Immunodeficiency Virus, Respiratory Syncytial Virus, Eppstein-Barr Virus, Influenza Virus, Cytomegalovirus, Herpes-Virus and Severe Acute Respiratory Syndrome.

### DEFINITIONS

Prior to a discussion of the detailed embodiments of the invention, a definition of specific terms related to the main aspects of the invention is provided.

In the context of the present invention IL-21 is defined as the sequence disclosed in WO00/53761 as SEQ ID No.:2. The invention also embraces DNA sequences encoding the peptide as SEQ ID No. 1, functional derivatives and fragments thereof. The present application also describes analogues of IL-21 and derivatives thereof. In the context of the present invention the term "IL-21" thus means IL-21 as described in WO00/53761, while "IL-21 and derivatives thereof" covers as well IL-21 as variants, analogues and derivatives thereof, accordingly.

In accordance with the present invention there may be employed conventional molecular biology, microbiology, and recombinant DNA techniques within the skill of the art. Such techniques are explained fully in the literature. See, e.g., Sambrook, Fritsch & Maniatis, Molecular Cloning: A Laboratory Manual, Second Edition (1989) Cold Spring Harbor Laboratory Press, Cold Spring Harbor, New York (herein "Sambrook et al., 1989") DNA Cloning: A Practical Approach, Volumes I and II/D.N. Glover ed. 1985); Oligonucleotide Synthesis (M.J. Gait ed. 1984); Nucleic Acid Hybridization (B.D. Hames & S.J. Higgins eds (1985)); Transcription And Translation (B.D. Hames & S.J. Higgins, eds. (1984)); Animal Cell Culture (R.I. Freshney, ed. (1986)); Immobilized Cells And Enzymes (IRL Press, (1986)); B. Perbal, A Practical Guide To Molecular Cloning (1984).

An "effective amount" means an amount that is sufficient to provide a clinical effect. It will depend on the means of administration, target site, state of the patient, whether the treatment takes place in the subject or on isolated cells, the frequency of treatment etc. Dosage ranges would ordinarily be expected from 0.1 microgram to 3000 microgram per kilogram of body weight per day. For a complete discussion of drug formulations and dosage ranges see Remington's Pharmaceutical Sciences, 18th Ed., (Mack Publishing Co., Easton, Penn., 1996).

It is to be understood that this invention is not limited to the particular methodology, protocols and reagents described, as such may vary. It is also understood that the terminology used herein is for the purpose of describing particular embodiments only, and is not intended to limit the scope of the present invention.

Those of skill will readily appreciate that dose levels can vary as a function of the specific compound, the severity of the symptoms and the susceptibility of the subject to side effects. Preferred dosages for a given compound are readily determinable to those of skilled in the art by a variety of means. A preferred means is to measure the physiological potency of a given compound.

In the context of the present invention "administration", "combined administration" or "combination therapy" refers to a treatment of cancer or viral infections by administering IL-21, an analogue or derivative thereof and any agent or combination of agents that induce cell death and/or any agent or combination of agents that enhance the immune response and/or any agent or combination of agents that interfere with tumor growth, metastases or spread of cancer or virus-infected cells. Said combination therapy can be performed by administering IL-21, an analogue or derivative thereof prior to said agents or combination of agents and/or by simultaneous administration of IL-21, an analogue or a derivative thereof and said agents or combination of agents and/or by administration of IL-21, an analogue or a derivative thereof after administration of said agents or combination of agents.

In the context of the present invention "treatment" or "treating" refers to preventing, alleviating, managing, curing or reducing the disease.

In the context of the present invention "cancer" refers to any neoplastic disorder, including such cellular disorders as for example sarcoma, carcinoma, melanoma, leukemia, lymphoma, cancers in the breast, head and neck, ovaries, bladder, lung, pharynx, larynx, oesophagus, stomach, small intestines, liver, pancreas, colon, female reproductive tract, male reproductive tract, prostate, kidneys and central nervous system.

In the context of the present invention the combinations provide an "effective amount" as applied to IL-21, an analogue or a derivative thereof or any of the combinations and refers to the amount of each component of the mixture which is effective for survival of the host.

Synergy can be measured in terms of dose, survival, time to progression, disease free period, reduced tumor burden or other parameters suitable for the disease.

### DESCRIPTION OF THE INVENTION

"IL-21" is described in International Patent Application publication no. WO 00/53761, published September 14, 2000, which is hereby incorporated in this application in its entirety, discloses IL-21 (as "Zalpha11 ligand") as SEQ ID No. 2, which is hereby incorporated in this application in its entirety, as well as methods for producing it and antibodies thereto and a polynucleotide sequence encoding IL-21 as SEQ ID No. 1 in the aforementioned application. The invention comprises their orthologs comprising at least 70%, at least 80%, at least 90%, at least 95%, or greater than 95% sequence identity. The present invention also includes the use of polypeptides that comprise an amino acid sequence having at least 70%, at least 80%, at least 90%, at least 95% or greater than 95% sequence identity to the sequence of amino acid residues 1 to 162, residues 41(Gln) to 148(Ile) of SEQ ID No: 2. Methods for determining percent identity are described below. The IL-21 polypeptides of the present invention have retained all or some of the biological activity of IL-21 which makes IL-21 useful for treating for example infections and cancer. Some of the polypeptides may also have a biological activity which is higher than the biological activity of IL-21.
The present invention embraces counterpart proteins and polynucleotides from other species ("orthologs"). Of particular interest are IL-21 polypeptides from other mammalian species, including rodent, porcine, ovine, bovine, canine, feline, equine, and other primates. Species orthologs of the human IL-21 protein can be cloned using information and compositions provided by the present invention in combination with conventional cloning techniques. As used and claimed, the language "an isolated polynucleotide which encodes a polypeptide, said polynucleotide being defined by SEQ ID NOs: 2" includes all allelic variants and species orthologs of this polypeptide.

The present invention also provides isolated protein polypeptides that are substantially identical to the protein polypeptide of SEQ ID NO: 2 and its species orthologs. By "isolated" is meant a protein or polypeptide that is found in a condition other than its native environment, such as apart from blood and animal tissue. In a preferred form, the isolated polypeptide is substantially free of other polypeptides, particularly other polypeptides of animal origin. It is preferred to provide the polypeptides in a highly purified form, i.e. greater than 95% pure, more preferably greater than 99% pure. The term "substantially identical" is used herein to denote polypeptides having more then 50%, preferably more then 60%, more then 70% or more preferably at least 80%, sequence identity to the sequence shown in SEQ ID NOs: 2 of WO00/53761 or species orthologs. Such polypeptides will more preferably be at least 90% identical, and most preferably at least 95% or more identical to IL21, or its species orthologs. Percent sequence identity is determined by conventional methods. See, for example, Altschul et al., Bull. Math. Bio. 48: 603-616 (1986) and Henikoff and Henikoff, Proc. Natl. Acad. Sci. USA 89:10915-10919 (1992). Sequence identity of polynucleotide molecules is determined by similar methods using a ratio as disclosed above.

Variant IL-21 polypeptides or as also used herein, IL-21 analogues, are substantially identical proteins and polypeptides and are characterized as having one or more amino acid substitutions, deletions or additions. These changes are preferably of a minor nature, that is conservative amino acid substitutions (see Table 1) and other substitutions that do not significantly affect the folding or activity of the protein or polypeptide; small deletions, typically of one to about 30 amino acids; and small amino- or carboxyl-terminal extensions, such as an amino-terminal methionine residue, a small linker peptide of up to about 20-25 residues, or a small extension that facilitates purification (an affinity tag), such as a poly- histidine tract, protein A, Nilsson et al., EMBO J. 4:1075 (1985); Nilsson et al., Methods Enzymol. 198:3 (1991), glutathione S transferase, Smith and Johnson, Gene 67:31 (1988), or other antigenic epitope or binding domain. See, in general Ford et al., Protein Expression and Purification 2: 95-107 (1991). DNAs encoding affinity tags are available from commercial suppliers (e.g., Pharmacia Biotech, Piscataway, NJ).

**Table 1**

| Conservative amino acid substitutions | |
|---|---|
| Basic: | arginine |
| | lysine |
| | histidine |
| Acidic: | glutamic acid |
| | aspartic acid |
| Polar: | glutamine |
| | asparagine |
| Hydrophobic: | leucine |
| | isoleucine |
| | valine |
| Aromatic: | phenylalanine |
| | tryptophan |
| | tyrosine |
| Small: | glycine |
| | alanine |
| | serine |
| | threonine |
| | methionine |

The proteins of the present invention can also comprise non-naturally occurring amino acid residues. Non-naturally occurring amino acids include, without limitation, trans-3-methylproline, 2,4-methanoproline, cis-4-hydroxyproline, trans-4-hydroxyproline, N-methylglycine, addo-threonine, methylthreonine, hydroxyethylcysteine, hydroxyethylhomocysteine, nitroglutamine, homoglutamine, pipecolic acid, thiazolidine carboxylic acid, dehydroproline, 3- and 4-methylproline, 3,3-dimethylproline, tert-leucine, norvaline, 2-azaphenylalanine, 3-azaphenylalanine, 4-azaphenylalanine, and 4-fluorophenylalanine. Several methods are known in the art for incorporating nonnaturally occurring amino acid residues into proteins. For example, an in vitro system can be employed wherein nonsense mutations are suppressed using chemically aminoacylated suppressor tRNAs. Methods for synthesizing amino acids and aminoacylating tRNA are known in the art. Essential amino acids in the polypeptides of the present invention can be identified according to procedures known in the art, such as site-directed mutagenesis or alaninescanning mutagenesis [Cunningham and Wells, Science 244: 1081-1085 (1989)]; Bass et al., Proc. Natl. Acad. Sci. USA 88:4498-4502 (1991). In the latter technique, single alanine mutations are introduced at every residue in the molecule, and the resultant mutant molecules are tested for biological activity (e.g., ligand binding and signal transduction) to identify amino acid residues that are critical to the activity of the molecule. Sites of ligand-protein interaction can also be determined by analysis of crystal structure as determined by such techniques as nuclear magnetic resonance, crystallography or photoaffinity labeling.

Multiple amino acid substitutions can be made and tested using known methods of mutagenesis and screening, such as those disclosed by Reidhaar-Olson and Sauer, Science 241:53-57 (1988) or Bowie and Sauer Proc. Natl. Acad. Sci. USA 86:2152-2156 (1989). Briefly, these authors disclose methods for simultaneously randomizing two or more positions in a polypeptide, selecting for functional polypeptide, and then sequencing the mutagenized polypeptides to determine the spectrum of allowable substitutions at each position. Other methods that can be used include phage display (e.g., Lowman et al., Biochem.30:10832-10837 (1991); Ladner et al., U.S. Patent No. 5,223,409; Huse, WIPO Publication WO 92/06204) and region-directed mutagenesis, Derbyshire et al., Gene 46:145 (1986); Ner et al., DNA 7:127 (1988).
Variants of IL-21 is for example the IL-21 peptide as described above, without the N-terminal sequence. The N-terminal sequence may comprise the initial1-28 amino acids. Also or alternatively a variant is for example IL-21 or IL-21 variants without the N-terminal Met, which is often included from the expression in a bacterial host.
Mutagenesis methods as disclosed above can be combined with high-throughput screening methods to detect activity of cloned, mutagenized proteins in host cells. Preferred assays in this regard include cell proliferation assays and biosensor-based ligand-binding assays, which are described below. Mutagenized DNA molecules that encode active proteins or portions thereof (e.g., ligand-binding fragments) can be recovered from the host cells and rapidly sequenced using modern equipment. These methods allow the rapid determination of the importance of individual amino acid residues in a polypeptide of interest, and can be applied to polypeptides of unknown structure.

The present invention further provides a variety of other polypeptide fusions and related multimeric proteins comprising one or more polypeptide fusions. For example, a IL-21 polypeptide can be prepared as a fusion to a dimerizing protein as disclosed in U.S. Patents Nos. 5,155,027 and 5,567,584. Preferred dimerizing proteins in this regard include immunoglobulin constant region domains. Immunoglobulin-IL-21 polypeptide fusions can be expressed in genetically engineered cells Auxiliary domains can be fused to IL-21 polypeptides to target them to specific cells, tissues, or macromolecules (e.g., collagen). For example, a IL-21 polypeptide or protein could be targeted to a predetermined cell type by fusing a polypeptide to a ligand that specifically binds to a receptor on the surface of the target cell. In this way, polypeptides and proteins can be targeted for therapeutic or diagnostic purposes. A IL-21 polypeptide can be fused to two or more moieties, such as an affinity tag for purification and a targeting domain. Polypeptide fusions can also comprise one or more cleavage sites, particularly between domains. See, Tuan et al., Connective Tissue Research 34:1-9 (1996).

"IL-21 derivatives" comprises derivatisation or linking to another functional molecule. The linking can be chemical coupling, genetic fusion, non-covalent association or the like, to other molecular entities such as antibodies, toxins, radioisotope, cytotoxic or cytostatic agents or polymeric molecules or lipophilic groups. Non-limiting examples include polymeric groups such as, e.g, dendrimers as disclosed in PCT/DK2004/000531, polyalkylene oxide (PAO), polyalkylene glycol (PAG), polyethylene glycol (PEG), polypropylene glycol (PPG), branched PEGs, polyvinyl alcohol (PVA), polycarboxylate, poly-vinylpyrolidone, polyethylene-co-maleic acid anhydride, polystyrene-co-maleic acid anhydride; dextran, carboxymethyl-dextran; serum protein binding-ligands, such as compounds which bind to albumin, like fatty acids, C₅-C₂₄ fatty acid, aliphatic diacid (e.g. C₅-C₂₄). Albumin binders are described in Danish patent applications PCT/DK04/000625. Albumin binders are also compounds of the following formula:

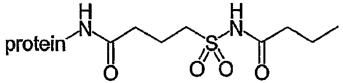

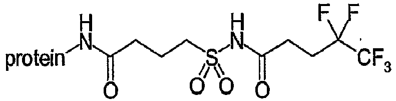

Other examples of protracting groups includes small organic molecules containing moieties that under physiological conditions alters charge properties, such as carboxylic acids or amines, or neutral substituents that prevent glycan specific recognition such as smaller alkyl substituents (e.g., C₁-C₅ alkyl).
The term "polymeric molecule", or "polymeric group" or "polymeric moiety" or "polymer molecule", encompasses molecules formed by covalent linkage of two or more monomers wherein none of the monomers is an amino acid residue. Preferred polymers are polymer molecules selected from the group consisting of dendrimers as disclosed in PCT/DK2004/000531, polyalkylene oxide (PAO), including polyalkylene glycol (PAG), such as polyethylene glycol (PEG) and polypropylene glycol (PPG), branched PEGs, polyvinyl alcohol (PVA), polycarboxylate, poly-vinylpyrolidone, polyethylene-co-maleic acid anhydride, polystyrene-co-maleic acid anhydride, and dextran, including carboxymethyl-dextran, PEG being particularly preferred.

The term "PEGylated IL-21 "means IL-21, having one or more PEG molecule conjugated to a human IL-21 polypeptide. It is to be understood, that the PEG molecule may be attached to any part of the IL-21 polypeptide including any amino acid residue or carbohydrate moiety of the IL-21 polypeptide. The term "cysteine-PEGylated IL-21 "means IL-21 having a PEG molecule conjugated to a sulfhydryl group of a cysteine introduced in IL-21.
The term "polyethylene glycol" or "PEG" means a polyethylene glycol compound or a derivative thereof, with or without coupling agents, coupling or activating moeities (e.g., with thiol, triflate, tresylate, azirdine, oxirane, or preferably with a maleimide moiety). Compounds such as maleimido monomethoxy PEG are exemplary of activated PEG compounds of the invention. The term "PEG" is intended to indicate polyethylene glycol of a molecular weight between 500 and 150,00ODa, including analogues thereof, wherein for instance the terminal OH-group has been replaced by a methoxy group (referred to as mPEG).
PEG is a suitable polymer molecule, since it has only few reactive groups capable of cross-linking compared to polysaccharides such as dextran. In particular, monofunctional PEG, e.g. methoxypolyethylene glycol (mPEG), is of interest since its coupling chemistry is relatively simple (only one reactive group is available for conjugating with attachment groups on the polypeptide). Consequently, the risk of cross-linking is eliminated, the resulting polypeptide conjugates are more homogeneous and the reaction of the polymer molecules with the polypeptide is easier to control.

To effect covalent attachment of the polymer molecule(s) to the polypeptide, the hydroxyl end groups of the polymer molecule are provided in activated form, i.e. with reactive functional groups. Suitable activated polymer molecules are commercially available, e.g. from Shearwater Corp., Huntsville, Ala., USA, or from PolyMASC Pharmaceuticals plc, UK. Alternatively, the polymer molecules can be activated by conventional methods known in the art, e.g., as disclosed in WO 90/13540. Specific examples of activated linear or branched polymer molecules for use in the present invention are described in the Shearwater Corp. 1997 and 2000 Catalogs (Functionalized Biocompatible Polymers for Research and pharmaceuticals, Polyethylene Glycol and Derivatives, incorporated herein by reference). Specific examples of activated PEG polymers include the following linear PEGs: NHS-PEG (e.g. SPA-PEG, SSPA-PEG, SBA-PEG, SS-PEG, SSA-PEG, SC-PEG, SG-PEG, and SCM-PEG), and NOR-PEG), BTC-PEG, EPOX-PEG, NCO-PEG, NPC-PEG, CDI-PEG, ALD-PEG, TRES-PEG, VS-PEG, IODO-PEG, and MAL-PEG, and branched PEGs such as PEG2-NHS and those disclosed in U.S. Pat. No. 5,932,462 and U.S. Pat. No. 5,643,575, both of which are incorporated herein by reference. Furthermore, the following publications, incorporated herein by reference, disclose useful polymer molecules and/or PEGylation chemistries: U.S. Pat. No. 5,824,778, U.S. Pat. No. 5,476,653, WO 97/32607, EP 229,108, EP 402,378, U.S. Pat. No. 4,902,502, U.S. Pat. No. 5,281,698, U.S. Pat. No. 5,122,614, U.S. Pat. No. 5,219,564, WO 92/16555, WO 94/04193, WO 94/14758, WO 94/17039, WO 94/18247, WO 94/28024, WO 95/00162, WO 95/11924, WO 95/13090, WO 95/33490, WO 96/00080, WO 97/18832, WO 98/41562, WO 98/48837, WO 99/32134, WO 99/32139, WO 99/32140, WO 96/40791, WO 98/32466, WO 95/06058, EP 439 508, WO 97/03106, WO 96/21469, WO 95/13312, EP 921 131, U.S. Pat. No. 5,736,625, WO 98/05363, EP 809 996, U.S. Pat. No. 5,629,384, WO 96/41813, WO 96/07670, U.S. Pat. No. 5,473,034, U.S. Pat. No. 5,516,673, EP 605 963, U.S. Pat. No. 5,382,657, EP 510 356, EP 400 472, EP 183 503 and EP 154 316.
The conjugation of the polypeptide and the activated polymer molecules is conducted by use of any conventional method, e.g. as described in the following references (which also describe suitable methods for activation of polymer molecules): R. F. Taylor, (1991), "Protein immobilisation. Fundamental and applications", Marcel Dekker, N.Y.; S. S. Wong, (1992), "Chemistry of Protein Conjugation and Crosslinking", CRC Press, Boca Raton; G. T. Hermanson et al., (1993), "Immobilized Affinity Ligand Techniques", Academic Press, N.Y.). The skilled person will be aware that the activation method and/or conjugation chemistry to be used depends on the attachment group(s) of the polypeptide (examples of which are given further above), as well as the functional groups of the polymer (e.g. being amine, hydroxyl, carboxyl, aldehyde, sulfydryl, succinimidyl, maleimide, vinysulfone or haloacetate). The PEGylation may be directed towards conjugation to all available attachment groups on the polypeptide (i.e. such attachment groups that are exposed at the surface of the polypeptide) or may be directed towards one or more specific attachment groups, e.g. the N-terminal amino group (U.S. Pat. No. 5,985,265). Furthermore, the conjugation may be achieved in one step or in a stepwise manner (e.g. as described in WO 99/55377).
It will be understood that the PEGylation is designed so as to produce the optimal molecule with respect to the number of PEG molecules attached, the size and form of such molecules (e.g. whether they are linear or branched), and where in the polypeptide such molecules are attached. The molecular weight of the polymer to be used will be chosen taking into consideration the desired effect to be achieved. For instance, if the primary purpose of the conjugation is to achieve a conjugate having a high molecular weight and larger size (e.g. to reduce renal clearance), one may choose to conjugate either one or a few high molecular weight polymer molecules or a number of polymer molecules with a smaller molecular weight to obtain the desired effect. Preferably, however, several polymer molecules with a lower molecular weight will be used. This is also the case if a high degree of epitope shielding Is desired.
In such cases, 2-8 polymers with a molecular weight of e.g. about 5,000 Da, such as 3-6 such polymers, may for example be used. As the examples below illustrate, it may be advantageous to have a larger number of polymer molecules with a lower molecular weight (e.g. 4-6 with a MW of 5000) compared to a smaller number of polymer molecules with a higher molecular weight (e.g. 1-3 with a MW of 12,000-20,000) in terms of improving the functional in vivo half-life of the polypeptide conjugate, even where the total molecular weight of the attached polymer molecules in the two cases is the same or similar. It is believed that the presence of a larger number of smaller polymer molecules provides the polypeptide with a larger diameter or apparent size than e.g. a single yet larger polymer molecule, at least when the polymer molecules are relatively uniformly distributed on the polypeptide surface.
It has further been found that advantageous results are obtained when the apparent size (also referred to as the "apparent molecular weight" or "apparent mass") of at least a major portion of the conjugate of the invention is at least about 50 kDa, such as at least about 55 kDa, such as at least about 60 kDa, e.g. at least about 66 kDa. This is believed to be due to the fact that renal clearance is substantially eliminated for conjugates having a sufficiently large apparent size. In the present context, the "apparent size" of a IL-21 conjugate or IL-21 polypeptide is determined by the SDS-PAGE method.

In an embodiment of the invention PEG is conjugated to a peptide according to the present invention may be of any molecular weight. In particular the molecular weight may be between 500 and 100,000 Da, such as between 500 and 60,000 Da, such as between 1000 and 40,000 Da, such as between 5,000 and 40,000 Da. In particular, PEG with molecular weights of 10,000 Da, 20,000 Da or 40,000 KDa may be used in the present invention. In all cases the PEGs may be linear or branched. In an embodiment of the invention the PEG groups are 5kDa, 10kDa, 20kDa, 30kDa, 40kDa og 60kDa.
In an embodiment of the invention, one or more polymeric molecules are added to IL-21 or an analogue thereof.
The term "lipophilic group" is characterised by comprising 4-40 carbon atoms and having a solubility in water at 20°C in the range from about 0.1 mg/100 ml water to about 250 mg/100 ml water, such as in the range from about 0.3 mg/100 ml water to about 75 mg/100 ml water. For instance, octanoic acid (C8) has a solubility in water at 20°C of 68 mg/100 ml, decanoic acid (C10) has a solubility in water at 20°C of 15 mg/100 ml, and octadecanoic acid (C18) has a solubility in water at 20°C of 0.3 mg/100 ml.
To obtain a satisfactory protracted profile of action of the IL-21 derivative, the lipophilic substituent attached to the IL-21 moiety, as an example comprises 4-40 carbon atoms, such as 8-25 carbon atoms. The lipophilic substituent may be attached to an amino group of the IL-21 moiety by means of a carboxyl) group of the lipophilic substituent which forms an amide bond with an amino group of the amino acid to which it is attached. As an alternative, the lipophilic substituent may be attached to said amino acid in such a way that an amino group of the lipophilic substituent forms an amide bond with a carboxyl group of the amino acid. As a further option, the lipophililic substituent may be linked to the IL-21 moiety via an ester bond. Formally, the ester can be formed either by reaction between a carboxyl group of the IL-21 moiety and a hydroxyl group of the substituent-to-be or by reaction between a hydroxyl group of the IL-21 moiety and a carboxyl group of the substituent-to-be. As a further alternative, the lipophilic substituent can be an alkyl group which is introduced into a primary amino group of the IL-21 moiety In one embodiment of the invention the IL-21 derivative only has one lipophilic substituent attached to the IL-21 peptide.
In one embodiment of the invention the lipophilic substituent comprises from 4 to 40 carbon atoms.
In one embodiment of the invention the lipophilic substituent comprises from 8 to 25 carbon atoms.
In one embodiment of the invention the lipophilic substituent comprises from 12 to 20 carbon atoms.
In one embodiment of the invention the lipophilic substituent is attached to an amino acid residue in such a way that a carboxyl group of the lipophilic substituent forms an amide bond with an amino group of the amino acid residue.
In other preferred embodiments, additional lysines are substituted, inserted into the sequence or added at the N-terminal or C-terminal, and then optionally derivatised.
Preferred regions of insertions are where the overall activity of the protein is not adversely affected.

In one embodiment of the invention the lipophilic substituent is attached to an amino acid residue in such a way that an amino group of the lipophilic substituent forms an amide bond with a carboxyl group of the amino acid residue.
In one embodiment of the invention the lipophilic substituent is attached to the IL-21 peptide by means of a spacer.
In one embodiment of the invention the spacer is an unbranched alkane α,ω-dicarboxylic acid group having from 1 to 7 methylene groups, such as two methylene groups which spacer forms a bridge between an amino group of the IL-21 peptide and an amino group of the lipophilic substituent.

In one embodiment of the invention the spacer is an amino acid residue except a Cys residue, or a dipeptide. Examples of suitable spacers includes β-alanine, gamma-aminobutyric acid (GABA), γ-glutamic acid, succinic acid, Lys, Glu or Asp, or a dipeptide such as Gly-Lys. When the spacer is succinic acid, one carboxyl group thereof may form an amide bond with an amino group of the amino acid residue, and the other carboxyl group thereof may form an amide bond with an amino group of the lipophilic substituent. When the spacer is Lys, Glu or Asp, the carboxyl group thereof may form an amide bond with an amino group of the amino acid residue, and the amino group thereof may form an amide bond with a carboxyl group of the lipophilic substituent. When Lys is used as the spacer, a further spacer may in some instances be inserted between the ε-amino group of Lys and the lipophilic substituent. In one embodiment, such a further spacer is succinic acid which forms an amide bond with the ε-amino group of Lys and with an amino group present in the lipophilic substituent. In another embodiment such a further spacer is Glu or Asp which forms an amide bond with the ε-amino group of Lys and another amide bond with a carboxyl group present in the lipophilic substituent, that is, the lipophilic substituent is a N^{ε}-acylated lysine residue.
In one embodiment of the invention the spacer is selected from the list consisting of β-alanine, gamma-aminobutyric acid (GABA), γ-glutamic acid, Lys, Asp, Glu, a dipeptide containing Asp, a dipeptide containing Glu, or a dipeptide containing Lys. In one embodiment of the invention the spacer is β-alanine. In one embodiment of the invention the spacer is gamma-aminobutyric acid (GABA). In one embodiment of the invention the spacer is γ-glutamic acid.
In one embodiment of the invention a carboxyl group of the parent IL-21 peptide forms an amide bond with an amino group of a spacer, and the carboxyl group of the amino acid or dipeptide spacer forms an amide bond with an amino group of the lipophilic substituent.
In one embodiment of the invention an amino group of the parent IL-21 peptide forms an amide bond with a carboxylic group of a spacer, and an amino group of the spacer forms an amide bond with a carboxyl group of the lipophilic substituent.
In one embodiment of the invention the lipophilic substituent comprises a partially or completely hydrogenated cyclopentanophenathrene skeleton.
In one embodiment of the invention the lipophilic substituent is an straight-chain or branched alkyl group. In one embodiment of the invention the lipophilic substituent is the acyl group of a straight-chain or branched fatty acid.
In one embodiment of the invention the acyl group of a lipophilic substituent is selected from the group comprising CH₃(CH₂)ₙCO-, wherein n is 4 to 38, such as CH₃(CH₂)₆CO-, CH₃(CH₂)₈CO-, CH₃(CH₂)₁₀CO-, CH₃(CH₂)₁₂CO-, CH₃(CH₂)₁₄CO-, CH₃(CH₂)₁₆CO-, CH₃(CH₂)₁₈CO-, CH₃(CH₂)₂₀CO- and CH₃(CH₂)₂₂CO-.
In one embodiment of the invention the lipophilic substituent is an acyl group of a straight-chain or branched alkane α,ω-dicarboxylic acid.
In one embodiment of the invention the acyl group of the lipophilic substituent is selected from the group comprising HOOC(CH₂)ₘCO-, wherein m is 4 to 38, such as HOOC(CH₂)₁₄CO-, HOOC(CH₂)₁₆CO-, HOOC(CH₂)₁₈CO-, HOOC(CH₂)₂₀CO- and HOOC(CH₂)₂₂CO-.
In one embodiment of the invention the lipophilic substituent is a group of the formula CH₃(CH₂)ₚ((CH₂)_{q}COOH)CHNH-CO(CH₂)₂CO-, wherein p and q are integers and p+q is an integer of from 8 to 40, such as from 12 to 35.
In one embodiment of the invention the lipophlic substituent is a group of the formula CH₃(CH₂)ᵣCO-NHCH(COOH)(CH₂)₂CO-, wherein r is an integer of from 10 to 24.
In one embodiment of the invention the lipophilic substituent is a group of the formula CH₃(CH₂)ₛCO-NHCH((CH₂)₂COOH)CO-, wherein s is an integer of from 8 to 24.
In one embodiment of the invention the lipophilic substituent is a group of the formula COOH(CH₂)ₜCO- wherein t is an integer of from 8 to 24.
In one embodiment of the invention the lipophilic substituent is a group of the formula -NHCH(COOH)(CH₂)₄NH-CO(CH₂)ᵤCH₃, wherein u is an integer of from 8 to 18.
In one embodiment of the invention the lipophilic substituent is a group of the formula -NHCH(COOH)(CH₂)₄NH-COCH((CH₂)₂COOH)NH-CO(CH₂)_{w}CH₃, wherein w is an integer of from 10 to 16.
In one embodiment of the invention the lipophilic substituent is a group of the formula -NHCH(COOH)(CH₂)₄NH-CO(CH₂)₂CH(COOH)NH-CO(CH₂)ₓCH₃, wherein x is an integer of from 10 to 16.
In one embodiment of the invention the lipophilic substituent is a group of the formula -NHCH(COOH)(CH₂)₄NH-CO(CH₂)₂CH(COOH)NHCO(CH₂)_{y}CH₃, wherein y is zero or an integer of from 1 to 22.
In one embodiment of the invention the lipophilic substituent is N-Lithocholoyl.
In one embodiment of the invention the lipophilic substituent is N-Choloyl.
In one embodiment of the invention the IL-21 derivative has one lipophilic substituent. In one embodiment of the invention the IL-21 derivative has two lipophilic substituents. In one embodiment of the invention the IL-21 derivative has three lipophilic substituents. In one embodiment of the invention the IL-21 derivative has four lipophilic substituents.

In the present context, the words "peptide" and "polypeptide" and "protein" are used interchangeably and are intended to indicate the same.

IL-21 and variants thereof may be expressed in E-coli as described in WO 04/55168. Optionally IL-21 variants may be produced by recombinant DNA techniques in other organismes. To this end, DNA sequences encoding human IL-21 related polypeptides or IL-21 variants may be isolated by preparing a genomic or cDNA library and screening for DNA sequences coding for all or part of the protein by hybridization using synthetic oligonucleotide probes in accordance with standard techniques. For the present purpose, the DNA sequence encoding the protein is preferably of human origin, i.e, derived from a human genomic DNA or cDNA library.
The protein polypeptides of the present invention, including full-length proteins, protein fragments (e.g. ligand-binding fragments), and fusion polypeptides can be produced in genetically engineered host cells according to conventional techniques. Suitable host cells are those cell types that can be transformed or transfected with exogenous DNA and grown in culture, and include bacteria, fungal cells, and cultured higher eukaryotic cells. Eukaryotic cells, particularly cultured cells of multicellular organisms, are preferred. Techniques for manipulating cloned DNA molecules and introducing exogenous DNA into a variety of host cells are disclosed by Sambrook et al., Molecular Cloning: A Laboratory Manual, 2nd ed.(Cold Spring Harbor Laboratory Press, Cold Spring Harbor, NY, 1989), and Ausubel et al., ibid.

It is to be recognized that according to the present invention, when a cDNA is claimed as described above, it is understood that what is claimed are both the sense strand, the anti-sense strand, and the DNA as double-stranded having both the sense and anti-sense strand annealed together by their respective hydrogen bonds. Also claimed is the messenger RNA (mRNA) which encodes the polypeptides of the present invention, and which mRNA is encoded by the above-described cDNA. A messenger RNA (mRNA) will encode a polypeptide using the same codons as those defined above, with the exception that each thymine nucleotide (T) is replaced by a uracil nucleotide (U).
To direct an IL-21 polypeptide into the secretory pathway of a host cell, a secretory signal sequence (also known as a leader sequence, prepro sequence or pre sequence) is provided in the expression vector. The secretory signal sequence may be that of the protein, or may be derived from another secreted protein (e.g.,) or synthesized de novo. The secretory signal sequence is joined to the IL-21 DNA sequence in the correct reading frame. Secretory signal sequences are commonly positioned 5' to the DNA sequence encoding the polypeptide of interest, although certain signal sequences may be positioned elsewhere in the DNA sequence of interest (see, e. g., Welch et al., U.S. Patent No. 5,037,743; Holland et al., U.S.Patent No. 5,143,830).

Percentage sequence identity between two amino acid sequences is determined by a Needelman-Wunsch alignment, useful for both protein and DNA alignments. For protein alignments the default scoring matrix used is BLOSUM50, and the penalty for the first residue in a gap is -12, while the penalty for additional residues in a gap is -2. The alignment may be made with the Align software from the FASTA package version v20u6 (W.R. Pearson and D.J. Lipman (1988), "Improved Tools for Biological Sequence Analysis", PNAS 85:2444-2448; and W.R. Pearson (1990) "Rapid and Sensitive Sequence Comparison with FASTP and FASTA", Methods in Enzymology, 183:63-98).

In one embodiment the polypeptide used in the present invention is an isolated polypeptide. In another embodiment the polynucleotide used in the present invention is an isolated polynucleotide.

It is preferred to purify the polypeptides of the present invention to :>80% purity, more preferably to >90% purity, even more preferably >95% purity with respect to contaminating macromolecules, particularly other proteins and nucleic acids, and free of infectious and pyrogenic agents, and particularly preferred is a pharmaceutically pure state, that is greater than 98% pure or preferably greater than 99.9% pure with respect to contaminating macromolecules, particularly other proteins and nucleic acids, and free of infectious and pyrogenic agents. Preferably, a purified polypeptide is substantially free of other polypeptides, particularly other polypeptides of animal origin.

The following list of components or agents that can be used together with IL-21, or IL-21 analogues or derivatives thereof in combination therapy of cancer and viral infections is not intended in any way to limit the scope of the invention.

### I.

### a) Conventional chemotherapeutic agents

In one embodiment of the invention, combination therapy is performed by administering IL-21, analogues or derivatives thereof and conventional chemotherapic agents. Chemotherapeutic agents have different modes of actions such as by influencing either
a) DNA level
b) RNA level

Non-limiting examples of conventional chemotherapeutic agents at the DNA level or on the RNA level are anti-metabolites (such as Azathioprine, Cytarabine, Fludarabine phosphate, Fludarabine, Gemcitabine, cytarabine,Cladribine, Capecitabine 6-mercaptopurine, 6-thioguanine, methotrexate, 5-fluorouracil, and hydroxyurea) alkylating agents (such as Melphalan, Busulfan, Cis-platin, Carboplatin, Cyclophosphamide, Ifosphamide, Dacarbazine, procarbazine, Chlorambucil, Thiotepa, Lomustine, Temozolamide) anti-mitotic agents (such as Vinorelbine, Vincristine, Vinblastine, Docetaxel, Paclitaxel) topoisomerase inhibitors (such as Doxorubicin, Amsacrine, Irinotecan, Daunorubicin, Epirubicin, Mitomycin, Mitoxantrone, Idarubicin, Teniposide, Etoposide, Topotecan) antibiotics (such as actinomycin and bleomycin) asparaginase, or the anthracyclines or the taxanes.

In one embodiment of the invention, combination therapy is performed by administering IL-21 analogues or derivatives thereof, and dacarbazine (DTIC).

### b) Radiotherapy:

Certain tumors can be treated with radioation or radiopharmaceuticals. The source of radiation can be either external or internal to the patient being treated. When the source is external to the patient the therapy is known as external beam radiation therapy (EBRT). When the source of radiation is internal to the patient, the treatment is called brachytherapy (BT). Typical raioactive atoms that have been used include radium, Cesium-137, Iridium-192, Americium-241, Gold-198, Cobalt-57, Copper-67, Technetium-99, Iodide-123, Iodide-131 and Indium-111.
Radiation therapy is standard treatment to control unresectable or inoperable tumors and/or tumor metastases. Improved results have been seen when aradiation therapy has been combined with other therapies.

In an embodiment of the invention IL-21, an analogue or derivative thereof is administered in combination with radiation therapy.

MAbs have been developed for the treatment of leukaemia and lymphoma as well as solid tumor, and this principle is gaining increasing interest. These antibodies work either by inhibiting functions that are vital for survival of the tumor cells, by delivering a toxic payload, by interrupting key signalling events, or by induction of antibody-dependent cell-mediated cytotoxicity (ADCC) or complement-directed cytotoxicity (CDC) against the tumor cells. Death of the tumor cells might then lead to the release of tumor antigens that "vaccinates" the immune system and stimulates it to produce a secondary response that then targets the tumor cell (i.e. 'internal vaccination' as described below). Over-expressed oncogenes and tumor-specific antigens are key targets for many mAbs under development.

Tumor antigens are described for example in Stauss H, Kawakami Y and Parmiani G: Tumor antigens recognized by T cells and antibodies. Taylor and Frances (2003). The invention covers antibodies raised against these targets. The invention also covers antibodies raised against viral antigens.

In an embodiment of the invention IL-21, an analogue or derivative thereof is combined with the antibodies such as Rituximab, Alemtuzumab, Trastuzumab, Gemtuzumab, Gemtuzumab-ozogamicin (Myelotarg ®, Wyeth) Cetuximab (Erbitux™), Bevacizumab, HuMax-CD20, HuMax-EGFr, Zamyl and Pertuzumab.
In an embodiment of the invention IL-21, an analogue or derivative thereof is combined with Rituximab.
In an embodiment of the invention IL-21, an analogue or derivative thereof is combined with Cetuximab.
, In an embodiment of the invention IL-21 an analogue or derivative thereof is combined with an antibody against tissue factor, killer Ig-like receptors (KIR), laminin-5, EGF-R, VEGF-R, PDGF-R, HER-2/neu, or an antibody against a tumor antigen such as PSA, PSCA, CEA, CA125, KSA, etc.

In an embodiment of the invention, IL-21, an analogue or derivative thereof is administered together with a therapeutic antibody, such as those mentioned above, and further combined with additional ADCC-enhancing compounds, ex. blocking anti-KIR antibodies, NKG2D agonists, NKG2A antagonists, IL-2, IL-12, IL-15 or IL-21.

In another embodiment of the invention IL21 an analogue or derivative thereof is administered as a combination with antibodies against viral antigens.

### d) Cell cycle control/apoptosis regulators

A series of regulators are involved in the maintenance of normal cell-cycle. Compounds, which target regulators such as (i) cdc-25 (with NSC 663284 as a non-limiting example (Pu et al (2003) J Biol Chem 278, 46877)), (ii) cyclin-dependent kinases that overstimulate the cell cycle (with the following non-limiting examples: flavopiridol (L868275, HMR1275; Aventis), 7-hydroxystaurosporine (UCN-01, KW-2401; Kyowa Hakko Kogyo) and roscovitine (R-roscovitine, CYC202; Cyclacel) - as reviewed by Fischer & Gianella-Borradori (2003) Exp Op Invest Drugs 12, 955-970), and (iii) telomerase, the enzyme that helps cancer cells rebuild its telomeres are within the present invention such as the following non-limiting examples BIBR1532 (Damm et al (2001) EMBO J 20, 6958-6968) and SOT-095 (Tauchi et al (2003) Oncogene 22, 5338-5347). Furthermore, drugs that interfere with apoptotic pathways are within the present invention,such as the following non-limiting examples: TNF-related apoptosis-inducing ligand (TRAIL)/apoptosis-2 ligand (Apo-2L), antibodies that activate TRAIL receptors, IFNα and anti-sense Bcl-2.(see Igney and Krammer (2002) Nature Rev. Cancer 2, 277-288; Makin and Dive (2003) Trends Mol Med 9, 2519; Smyth et al (2003) Immunity 18, 1-6; Panaretakis et al (2003) Oncogene 22, 4543-4556 and references therein).. In one embodiment of the invention IL-21, an analogue or derivative thereof, is combined with one or more cell-cycle regulators and/or apoptosis-inducing agents.
In an embodiment of the invention above the compounds are selected from the group comprising cdc-25, NSC 663284, flavopiridol, 7-hydroxystaurosporine, roscovitine, BIBR1532 SOT-095, TNF-related apoptosis-inducing ligand (TRAIL)/apoptosis-2 ligand (Apo-2L), antibodies that activate TRAIL receptors, IFNα and anti-sense Bcl-2.

### e) Growth factor inhibitors

A number of mAbs against growth factors and growth factor receptors are being developed for the treatment of cancer. Thus, as a non-limiting example, members of the epidermal growth factor receptor (EGF-R) family are abnormally activated in many epithelial tumors, which often correlate with more aggressive clinical course. Antibodies directed against the extracellular ligand binding domain of these receptors and low molecular weight molecules that inhibit their tyrosine kinase domains are in late-stage clinical development or approved for treatment of cancer either as single agents or in combination with other cancer drugs. Non-limiting examples are Herceptin (monoclonal antibody), cetuximab (monoclonal antibody), Tarceva (low molecular weight inhibitor), and Iressa (low molecular weight inhibitor). In addition, the ligand can be neutralised before binding to the receptor.
In one embodiment of the invention IL-21, an analogue or derivative thereof is combined with growth factor inhibitors.

In an embodiment of the invention the growth factor inhibitors are selected from the group comprising Herceptin (monoclonal antibody), cetuximab (monoclonal antibody), Tarceva (low molecular weight inhibitor), and Iressa (low molecular weight inhibitor).

In an embodiment of the invention IL-21, an analogue or derivative thereof is combined with Herceptin.

### f) inhibitors of tumor vascularisation (Anti-angiogenesis drugs and anti-metastatic agents)

Tumor growth is dependent on sufficient blood supply and hence development of new blood vessels. This general feature of solid tumors seems attractive from a therapeutic point of view, i.e. reduced tumor growth and tumor regression is expected when treating patients wth cancer with anti-angionesis drugs. Currently, more than 60 anti-angionesis drugs are in clinical trials including the natural occurring endostatin and angiostatin (reviewed in Marx (2003) Science 301, 452-454). But also older chemotherapy drugs, other medicines and radiation therapy have anti-angiogenic effects. In one type of embodiments of the present invention is combination therapy with IL-21, analogues or derivatives thereof and one or more anti-angiogenic agents, such as the following non-limiting examples endostatin, angiostatin, antibodies that block factors that initiate angiogenesis (e.g. anti-VEGF - Avastin), low molecular compounds that inhibit angiogenesis by inhibiting key elements in relevant signal transduction pathways.

Attacking the vasculature of the tumor and the extracellular matrix has attracted increasing awareness. The following principles have so far been developed: Blockage of the endothelial cell, administration of angiostatin and endostatin, VEGF targeting and extracellular matrix In an embodiment of the invention IL-21, an analogue or derivative thereof is combined with an anti-angiogenesis drug.

In an embodiment of the invention the anti-angiogenesis drug is selected from the group comprising: avastin, neovastat, thalidomide, PTK787, ZK222584, ZD-6474 , SU6668, PD547,632, VEGF-Trap, CEP-7055, NM-3, SU11248. g) **Viral targeting** Viral targeting uses a recombinant virus - usually replication incompetent - to destroy a tumor directly. In practice, at least one round of replication occurs before the virus is incapacitated. Hence, the tumor is lysed, which often leads to systemic immunization with resulting protection. This approach has been refined further using genetic modification to enhance the immune response. For example, the genetic insertion of a human GM-CSF gene into a herpes simplex virus type 2 vector has been used improve the efficacy of the vaccine. In one embodiment of the invention, combination therapy is performed by administering IL-21, an analogue or a derivative thereof and viral targeting.

### h) Hormonal agents.

Hormonal agents are primarily know in the treatment of hormonal dependent cancers such as ovarian cancer, breast cancer and prostate cancer such as anti-androgen and anti-oestrogen therapy. Hormones and anti-hormones are compoundssuch as Estramustine phosphate, Polyestradiol phosphate, Estradiol, Anastrozole, Exemestane, Letrozole, Tamoxifen, Megestrol acetate, Medroxyprogesterone acetate, Octreotide, Cyproterone acetate, Bicaltumide, Flutamide, Tritorelin, Leuprorelin, Buserelin or Goserelin.

In an embodiment of the invention IL-21, an analogue or derivative thereof is combined with hormone therapy.

### II. Agents that enhance the immune response against tumor cells or virus-infected cells

j) The following list of components or agents that can be used together with IL-21, analogues or derivatives thereof in combination therapy of cancer and viral infections by enhancing the efficacy of the immune system is not intended in any way to limit the scope of the invention:

### Adjuvants:

Immunotherapy consist of specific and non-specific modalities. As examples of non-specific immunotherapy are adjuvants acting primarily as catalyst for the initiation of an immune response. Non-limiting examples of such vaccine adjuvants are QS21, GM-CSF and CpG oligodeoxynucleotides, lipopolysaccharide and polyinosinic:polycytidylic acid.

In one embodiment of the invention IL-21, an analogue or derivative thereof is combined with one or more adjuvants.

In an embodiment of the invention the adjuvants are selected from the group comprising: QS21, GM-CSF and CpG oligodeoxynucleotides, lipopolysaccharide and polyinosinic:polycytidylic acid, α-Galctosylceramide or analogues thereof, histamine dihydrochloride, or aluminum hydroxide.

### Cytokines

Non-limiting examples of cytokines are IFN-α IFN-β, IFN-γ, IL-2, PEG-IL-2, IL-4, IL-6, IL-7, IL-12, IL-13, IL-15, IL-18, IL-21, IL-23, IL-27, IL-28a, IL-28b, IL-29, GM-CSF, Flt3 ligand or stem cell factor.

In an embodiment of the invention IL-21, an analogue or derivative thereof is combined with one or more cytokines.

In an embodiment of the invention an IL-21 is combined with one or more of the compounds selected from the group comprising: IFN-α, IFN-β, IFN-γ, IL-2, PEG-IL-2, IL-4, IL-6, IL-7, IL-12, IL-13, IL-15, IL-18, IL-21, IL-23, IL-27, IL-28a, IL-28b, IL-29, GM-CSF, Flt3. ligand or stem cell factor, or an analogue or derivative of any of these.

In an embodiment of the invention, the compounds are selected from the group comprising : IFN-α, IFN-β, IFN-γ, PEG-IL-2, IL-18, IL-23, IL-27, IL-28a, IL-28b, IL-29. (In an embodiment of the invention IL-21, an analogue or derivative thereof is combined with one of the following: IL-2, PEG-IL-2, IL-7, IL-12, IL-15, and IFN-α. In an embodiment of the invention IL-21, an analogue or derivative thereof is combined with IL-12 In an embodiment of the invention IL-21, an analogue or derivative thereof is combined with IFN-α.
In an embodiment of the invention IL-21, an analogue or derivative thereof is combined with PEG-IL-2,
In an embodiment of the invention IL-21, an analogue or derivative thereof is combined with more than one of the following: IL-2, PEG-IL-2, IL-7, IL-12, IL-15, and IFN-α. In an embodiment of the invention IL-21, an analogue or derivative thereof is combined with at least one of the following: IL-2, PEG-IL-2, IL-7, IL-12, IL-15, and IFN-α and one additional active component. In an embodiment of the invention IL-21, an analogue or derivative thereof is combined with at least one of the following: IL-2, PEG-IL-2, IL-7, IL-12, IL-15, and IFN-α and one additional cytokine from the list above. In an embodiment of the invention IL-21, an analogue or derivative thereof is combined with IFN-α and GM-CSFIn an embodiment of the invention IL-21, an analogue or derivative thereof is combined with IFN-α and thymopentin. In an embodiment of the invention IL-21, an analogue or derivative thereof is combined with IFN-γ
In an embodiment of the invention IL-21, an analogue or derivative thereof is combined with autologous TILs and IFN-α
In an embodiment of the invention IL-21, an analogue or derivative thereof is combined with IFN-α and IL-12
In an embodiment of the invention IL-21, an analogue or derivative thereof is combined with Cis-platin and IFN-α
In an embodiment of the invention IL-21, an analogue or derivative thereof is combined with Cis-platin, tamoxifen and IFN-α
In an embodiment of the invention IL-21, an analogue or derivative thereof is combined with Cis-platin, DTIC, tamoxifen and IFN-α
In an embodiment of the invention IL-21, an analogue or derivative thereof is combined with Cis-platin, DTIC, tamoxifen and GM-CSF
In an embodiment of the invention IL-21, an analogue or derivative thereof is combined with Cis-platin, Carmustine, DTIC and IFN-α
In an embodiment of the invention IL-21, an analogue or derivative thereof is combined with Cis-platin, Carmustine, DTIC, tamoxifen and IFN-α
In an embodiment of the invention IL-21, an analogue or derivative thereof is combined with Cis-platin, Carmustine, DTIC, carboplatin, tamoxifen and IFN-α
In an embodiment of the invention IL-21, an analogue or derivative thereof is combined with Cis-platin, DTIC, Vinblastine, tamoxifen and IFN-α
In an embodiment of the invention IL-21, an analogue or derivative thereof is combined with Cis-platin, Vinblastine, temozolomide and IFN-α
In an embodiment of the invention IL-21, an analogue or derivative thereof is combined with Cis-platin, Carmustine, DTIC, Vindesine, tamoxifen and IFN-α
In an embodiment of the invention IL-21, an analogue or derivative thereof is combined with Cis-platin, tamoxifen and IFN-α
In an embodiment of the invention, IL-21, an analogue or derivative thereof is combined with Cis-platin, Vinblastine, DTIC and IFN-α
In an embodiment of the invention IL-21, an analogue or derivative thereof is combined with DTIC and IFN-α
In an embodiment of the invention IL-21, an analogue or derivative thereof is combined with DTIC, GM-CSF and IFN-α
In an embodiment of the invention IL-21, an analogue or derivative thereof is combined with DTIC, thymosin-α and IFN-α
In an embodiment of the invention IL-21, an analogue or derivative thereof is combined with Vinblastine and IFN-α
In an embodiment of the invention IL-21, an analogue or derivative thereof is combined with5-fluorouracil and IFN-α
In an embodiment of the invention IL-21, an analogue or derivative thereof is combined with Fotemustine and IFN-α
In an embodiment of the invention IL-21, an analogue or derivative thereof is combined with DTIC and autologous LAK cells
In an embodiment of the invention IL-21, an analogue or derivative thereof is combined with Gemcitabine and IFN-α
Any of the above combinations can be further combined with IL-2.

### Cellular immunotherapy

Examples of cellular immunotherapy (or adoptive immunotherapy) include reinfusion of ex-vivo expanded tumor infiltrating T cells or genetically modified T cells.

In one embodiment of the invention, combination therapy is combining administration of IL-21, an analogue or derivative thereof and cellular immunotherapy.

Cellular immunotherapy may include isolation of cells that can stimulate or exert an anti-cancer response from patients, expanding these into larger numbers, and reintroducing them into the same or another patient. In one aspect this may be CD4⁺ or CD8⁺ T cells recognizing tumor specific antigens or tumor-associated antigens. In another aspect this may be B cells expressing antibodies specific for tumor specific antigens or tumor-associated antigens. In another aspect this may be NK cells that are able to kill the tumor cells. In a preferred aspect this may be dendritic cells (DC) that are cultured ex vivo with a DC-expanding agent (e.g. GM-CSF or Flt3-L), loaded with tumor specific antigens or tumor-associated antigens and re-infused into a patient in need therepf. In one embodiment of the invention, combination therapy is combining administration of IL-21, an analogue or derivative thereof, and cellular immunotherapy or adoptive therapy.

In an embodiment of the invention the cell adoptive therapy comprises CD4⁺ or CD8⁺ T cells recognizing tumor specific antigens or tumor-associated antigens.

In an embodiment of the invention the cell adoptive therapy comprises B cell expressing antibodies specific for tumor specific antigens or tumor-associated antigens.

In an embodiment of the invention cell adoptive therapy comprises NK cells that are able to kill the tumor cells.

In an embodiment of the invention cell adoptive therapy comprises dendritic cells (DC).

In an embodiment of the above the dendritic cells are cultured in vivo with a DC expanding agent (e.g. GM-CSF or Flt3-L), loaded with tumor specific antigens or tumor-associated antigens and reintroduced in vivo.

### k) Agents that block inhibitory signalling in the immune system.

Immune responses, including anti-tumor and anti-viral responses, are regulated by a balance of signalling via stimulatory and inhibitory receptors in cells of the immune system. A shift towards abundant signalling via activatory receptors may lead to more effective immune responses, whereas enhanced signalling via inhibitory receptors may lead to less productive responses, or even may impair immunity. In order to enhance anti-tumor or anti-viral responses, it is useful to therapeutically block signalling via inhibitory receptors, in order to shift the balance towards activation. Therefore, agents that block inhibitory receptors, or inhibitory signalling pathways, are preferred agents for combination treatment, in conjunction with the IL-21, an analogue or derivative thereof. Non-limiting examples of such agents that block inhibitory receptors are mAbs specific for CTLA-4 (anti-CTLA-4), mAbs specific for KIR (anti-KIR), mAbs specific for LIR (anti-LIR), mAbs specific for CD94 (anti-CD94), or mAbs specific for NKG2A (anti-NKG2A).

Anti-anergic agents are small compounds, proteins, glycoproteins or antibodies that can break tolerance to tumor and cancer antigens.

Although the presence of tumor infiltrating lymphocytes (TILs) correlates with improved clinical outcome in a number of different cancer forms, there is clearly a need to improve the activity of these TILs due to anergy or tolerance to tumor antigens. The anergic condition may in a substantial number of cases be counteracted by monoclonal antibodies that prevent CTLA-4 -induced anergy or tolerance. Blockade of CTLA-4 has been shown in animal models, and in human cancer patients, to improve the effectiveness of cancer therapy suggesting that CTLA-4 blockade can be used to break the tolerance to cancer and tumor antigens. A non-limiting example of a monoclonal antibody that may be used for induction of the activity of TILs is MDX-010 (Phan et al. (2003) Proc. Natl. Acad. Sci. U.S.A. 100: 8372). In one embodiment of the invention, combination therapy is performed by administering IL-21, an analogue or derivative thereof and one or more agents that break the tolerance to cancer, tumor or viral antigens. In an embodiment of the invention IL-21, an analogue or derivative thereof is combined with MDX-010.

In an embodiment of the invention IL-21, an analogue or derivative thereof is combined with antibodies against CTLA-4.

In an embodiment of the invention IL-21, an analogue or derivative thereof is combined with antibodies against KIR.

In an embodiment of the invention IL-21, an analogue or derivative thereof is combined with antibodies against CD94.

In an embodiment of the invention IL-21, an analogue or derivative thereof is combined with antibodies against NKG2A.

In an embodiment of the invention IL-21, an analogue or derivative thereof is combined with antibodies against an inhibitory receptor expressed on an NK cell, a T cell or a NKT cell.

In an embodiment of the invention IL-21, an analogue or derivative thereof is combined with an antagonist of an inhibitory receptor.

In an embodiment of the invention IL-21, an analogue or derivative thereof is combined with an antagonist of a signalling protein involved in transmission of inhibitory signals.

### I) Therapeutic vaccines

The development of almost all human cancers involves genetic alterations, and this may lead to expression of altered molecules in tumor cells and over-expression of normal molecules, respectively. In principle, these changes should lead to an immune response from the host (immune surveillance). Obviously, this theoretical activation of the immune system only leads to spontaneous regression of the tumor in very few, exceptional cases. This may, among other factors, be due to lack of "danger signals", a phenomenon that has attracted increasing interest.

**Tumour specific** antigens have been identified, and vaccination with such antigens may stimulate the immune system to eradicate the tumor. Tumor-specific antigens (TSAs) are a relatively small group of antigens exemplified by the cancer-testis antigens. These genes are silent in normal tissue but are expressed by cancerous cells. They are highly specific markers of disease and include MAGE (melanoma antigen gene) found in melanoma.

**Tumor-associated antigens** (TAAs) are usually differentiation antigens expressed by normal cells but massively over-expressed in cancerous tissue. Targets initially thought to be specific for a particular cancer are actually quite common in many tumors, such as the gangliosides and mucin antigens. Classical differentiation antigens include MART-1 (melanoma antigen recognized by T cells) and gp 100, both from melanoma, tyrosinase, carcinoembryonic antigen (CEA) and gp75.

**Mutational antigens:** Point mutations are common in many cancers, and often occur in a similar location, such as the common mutation of the P53 or ras oncogenes. *In vitro* induction of human cytotoxic T-lymphocyte (CTL) responses against peptides of mutant and wild-type p53 has been reported. In a mouse model, mutant p53-pulsed dendritic cells were able to induce p53 specific CTL and inhibit the growth of established tumors.

**Viral antigens:** Certain viruses are oncogenic and gene products encoded by these viruses can elicit immune responses and thus serve as cancer antigens. An example is the E6 and E7 proteins from human papilloma virus type 16, which have been shown to induce cytotoxic T-lymphocyte responses *in vitro.*

Tumor-specific antigens, tumor-associated antigens and/or mutational antigens and viral antigens may be used either as peptides, recombinant purified single-agent antigens, combinations of recombinant purified antigens and/or purified or pools of antigens isolated from cancer cells or tumor cells as a vaccine to elicit an anti-tumor immune response. Similarly, peptides, recombinant purified single-agent antigens, combinations of recombinant antigens and/or purified or pools of antigens isolated from virus-infected cells may be used in a vaccine to elicit a response against virus-infected cells. Therapeutic vaccines can also be in the form of autologous tumor cell lysates or extracts, or lysates or extracts of allogeneic tumor cell-lines. Therapeutic vaccines can also be in the form of a DNA vaccine to elicit immune response against cancer and virus-infected cells. Said DNA vaccine may consist of an expression vector encoding the antigen alone or encoding the antigen together with a cytokine (eg. GM-CSF, IL-2, IL-12 or IL-21) that may enhance the immune response against cancer and virus-infected cells. Said DNA vaccine may also consist of a modified virus (eg. Fowlpox virus, Vaccinia virus or Adenovirus) that contains a DNA sequence encoding the antigen alone or encoding the antigen together with a cytokine. Therapeutic vaccines can also be in the form of anti-idiotype antibodies to elicit immune response against cancer and virus-infected cells. Therapeutic vaccines can also be in the form of autologous dendritic cells loaded with said antigens or peptides derived thereof together with a DC modifying agent, such as cytokines, toll-like receptor (TLR) agonists, CpG oligodeoxynucleotides, GM-CSF, or heat-shock proteins.

Said vaccine-mediated elicitation of an anti-tumor response or a response against virus-infected cells may be enhanced by administering adjuvants, cytokines, toll-like receptor (TLR) agonists, CpG oligodeoxynucleotides, dendritic cells, GM-CSF, or heat-shock proteins. In one embodiment of the invention, combination therapy is performed by administering IL-21, an analogue or derivative thereof with one or more therapeutic vaccines with or without adjuvants, cytokines, toll-like receptor (TLR) agonists, CpG oligodeoxynucleotides, dendritic cells, GM-CSF, or heat-shock proteins.

### Agents that interfere with tumor growth, metastasis or spread of virus-infected cells

n) Metastatic cancer cells penetrate the extracellular matrix (ECM) and the basement membrane of the blood vessels to metastasise to a target organ (ectopic site). EMC consists of proteins embedded in a carbohydrate complex (heparan sulfate peptidoglycans), and proteases surrounding the tumour are active in this breaking down the host tissue. Anti-metastatic agents antagonise the effect of such proteases (e.g. metalloproteinase inhibitors) (Coussens et al. Science 2002;295:2387-2392). In an embodiment of the present invention is combination therapy with IL-21, an analogue or a derivative thereof and one or more anti-metastatic agents, such as metalloproteinase inhibitors.

### IV. Internal vaccination

IV. "Internal vaccination" and "internal vaccination therapy" refer to drug- or radition-induced cell death of tumor cells that leads to elicitation of an immune response directed towards (i) said tumor cells as a whole or (ii) parts of said tumor cells including (a) secreted proteins, glycoproteins or other products, (b) membrane-associated proteins or glycoproteins or other components associated with or inserted in membranes and (c) intracellular proteins or other intracellular components. The immune response may be humoral (i.e. antibody - complement-mediated) or cell-mediated including but not limited to development of cytotoxic T lymphocytes that recognized said tumor cells or parts thereof. Internal vaccination bears many similarities to other vaccination procedures and involves many or all of the same cellular components of the hematopoietic and immune system with the advantage that the immunogens or antigenic components are endogenous and thus representative for the antigenic repertoire of said tumor cells. Internal vaccination may thus be considered personalized vaccination, which is elicited by use of general procedures for cancer treatment leading to tumor cell death. In addition to radiotherapy, non-limiting examples of drugs and agents that can be used to induce said tumor cell-death and internal vaccination are conventional chemotherapeutic agents, cell-cycle inhibitors, anti-angiogenesis drugs, monoclonal antibodies, apoptosis-inducing agents and signal transduction inhibitors.

"IL-21 and internal vaccination combination therapy" refers to combination therapy where IL-21, an analogue or derivative thereof is administered to patients with cancer who are treated with internal vaccination. IL-21, analogue or derivative may be administered prior to, concomitant with or after performing internal vaccination.

In an embodiment of the invention IL-21, an analogue or derivative thereof is included in an internal vaccination therapy.

Gene therapy includes transfer of genetic material into a cell to transiently or permanently alter the cellular phenotype. Different methods are investigated for delivery of cytokines, tumor antigens and additional stimulatory molecules. In the context of this invention IL-21 may be either the delivered agent or co-administered. In an embodiment of this invention IL-21 may be administered as a polynucleotide. The polynucleotide is described in WO 00/53761.

### VI. Immunosuppressive / immunomodulatory agents

### r) agents with influence on T-lymphocyte homing e.g. FTY-720

s) Calcineurin inhibitors such as valspodar, PSC 833, are active in preventing resistance development to cytotoxic agents due to inhibitory effects on MDR-1 and p-glycoprotein .

t) TOR-inhibitors act by blocking the serine-threonine kinase mammalian TOR (mTOR) Compounds such as sirolimus, everolimus and rapmycin are antiproliferative agents. They are involved in the downstream signaling cascades and are therefore relevante in the treatment of all tumour types (eg antiangiogenic properties).

### PHARMACEUTICAL COMPOSITIONS

IL-21 or other IL-21 polypeptides optionally together with the combination agent for use in treating cancer according to the present invention may be administered alone or in combination with pharmaceutically acceptable carriers or excipients, in either single or multiple doses. The formulation of the combination may be as one dose unit combining the compounds, or they may be formulated as seperate doses. The pharmaceutical compositions comprising IL-21 or other IL-21 polypeptides optionally together with the combination agent for use in treating cancer according to the present invention may be formulated with pharmaceutically acceptable carriers or diluents as well as any other known adjuvants and excipients in accordance with conventional techniques such as those disclosed in Remington: The Science and Practice of Pharmacy, 19th Edition, Gennaro, Ed., Mack Publishing Co., Easton, PA, 1995. The compositions may appear in conventional forms, for example capsules, tablets, aerosols, solutions or suspensions.

The pharmaceutical compositions may be specifically formulated for administration by any suitable route such as the oral, rectal, nasal, pulmonary, topical (including buccal and sublingual), transdermal, intracisternal, intraperitoneal, vaginal and parenteral (including subcutaneous, intramuscular, intra-tumoral, intrathecal, intravenous and intradermal) route. It will be appreciated that the preferred route will depend on the general condition and age of the subject to be treated, the nature of the condition to be treated and the active ingredient chosen. The route of administration may be any route, which effectively transports the active compound to the appropriate or desired site of action.

Pharmaceutical compositions for oral administration include solid dosage forms such as hard or soft capsules, tablets, troches, dragees, pills, lozenges, powders and granules. Where appropriate, they can be prepared with coatings such as enteric coatings or they can be formulated so as to provide controlled release of the active ingredient such as sustained or prolonged release according to methods well known in the art.

Liquid dosage forms for oral administration include solutions, emulsions, aqueous or oily suspensions, syrups and elixirs.

Pharmaceutical compositions for parenteral administration include sterile aqueous and non-aqueous injectable solutions, dispersions, suspensions or emulsions as well as sterile powders to be reconstituted in sterile injectable solutions or dispersions prior to use. Depot injectable formulations are also contemplated as being within the scope of the present invention.

Other suitable administration forms include suppositories, sprays, ointments, creams, gels, inhalants, dermal patches, implants etc.

A typical oral dosage is in the range of from about 0.001 to about 100 mg/kg body weight per day, such as from about 0.01 to about 50 mg/kg body weight per day, for example from about 0.05 to about 10 mg/kg body weight per day administered in one or more dosages such as 1 to 3 dosages. The exact dosage will depend upon the nature of the IL-21 or the IL-21 mimetic, together with the combination agent chosen, the frequency and mode of administration, the sex, age, weight and general condition of the subject treated, the nature and severity of the condition treated and any concomitant diseases to be treated and other factors evident to those skilled in the art.

The formulations may conveniently be presented in unit dosage form by methods known to those skilled in the art. A typical unit dosage form for oral administration one or more times per day such as 1 to 3 times per day may contain from 0.05 to about 1000 mg, for example from about 0.1 to about 500 mg, such as from about 0.5 mg to about 200 mg.

For parenteral routes such as intravenous, intrathecal, intramuscular, subcutaneous and similar administration, typical doses are in the order of about half the dose employed for oral administration.

Salts of IL-21 polypeptides are especially relevant when the protein is in solid or crystalline form

For parenteral administration, solutions of the IL-21 or the IL-21 mimetic, optionally together with the combination agent in sterile aqueous solution, aqueous propylene glycol or sesame or peanut oil may be employed. Such aqueous solutions should be suitably buffered if necessary and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art.

Suitable pharmaceutical carriers include inert solid diluents or fillers, sterile aqueous solution and various organic solvents. Examples of solid carriers are lactose, terra alba, sucrose, cyclodextrin, talc, gelatine, agar, pectin, acacia, magnesium stearate, stearic acid and lower alkyl ethers of cellulose. Examples of liquid carriers are syrup, peanut oil, olive oil, phospholipids, fatty acids, fatty acid amines, polyoxyethylene and water. Similarly, the carrier or diluent may include any sustained release material known in the art, such as glyceryl monostearate or glyceryl distearate, alone or mixed with a wax. The pharmaceutical compositions formed by combining an IL-21 or the IL-21 mimetic, optionally together with the combination agent for use in treating cancer according to the present invention and the pharmaceutically acceptable carriers are then readily administered in a variety of dosage forms suitable for the disclosed routes of administration. The formulations may conveniently be presented in unit dosage form by methods known in the art of pharmacy.

For nasal administration, the preparation may contain an IL-21 polypeptide or IL-21 mimetic dissolved or suspended in a liquid carrier, in particular an aqueous carrier, for aerosol application. The carrier may contain additives such as solubilizing agents, e.g. propylene glycol, surfactants, absorption enhancers such as lecithin (phosphatidylcholine) or cyclodextrin, or preservatives such as parabenes.

Formulations of IL-21 or the IL-21 mimetic, optionally together with the combination agent for use in treating cancer according to the present invention suitable for oral administration may be presented as discrete units such as capsules or tablets, each containing a predetermined amount of the active ingredient, and which may include a suitable excipient. Furthermore, the orally available formulations may be in the form of a powder or granules, a solution or suspension in an aqueous or non-aqueous liquid, or an oil-in-water or water-in-oil liquid emulsion.

Compositions intended for oral use may be prepared according to any known method, and such compositions may contain one or more agents selected from the group consisting of sweetening agents, flavouring agents, colouring agents, and preserving agents in order to provide pharmaceutically elegant and palatable preparations. Tablets may contain the active ingredient in admixture with non-toxic pharmaceutically-acceptable excipients which are suitable for the manufacture of tablets. These excipients may be for example, Inert diluents, such as calcium carbonate, sodium carbonate, lactose, calcium phosphate or sodium phosphate; granulating and disintegrating agents, for example com starch or alginic acid; binding agents, for example, starch, gelatine or acacia; and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in U.S. Patent Nos. 4,356,108; 4,166,452; and 4,265,874, incorporated herein by reference, to form osmotic therapeutic tablets for controlled release. Formulations for oral use may also be presented as hard gelatine capsules where the active ingredient is mixed with an inert solid diluent, for example, calcium carbonate, calcium phosphate or kaolin, or a soft gelatine capsules wherein the active ingredient is mixed with water or an oil medium, for example peanut oil, liquid paraffin, or olive oil.

Aqueous suspensions may contain the IL-21 or the IL-21 mimetic, optionally together with the combination agent in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide such as lecithin, or condensation products of an alkylene oxide with fatty acids, for example polyoxyethylene stearate, or condensation products of ethylene oxide with long chain aliphatic alcohols, for example, heptadecaethyl-eneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more colouring agents, one or more flavouring agents, and one or more sweetening agents, such as sucrose or saccharin.

Oily suspensions may be formulated by suspending the active ingredient in a vegetable oil, for example arachis oil, olive oil, sesame oil or coconut oil, or in a mineral oil such as a liquid paraffin. The oily suspensions may contain a thickening agent, for example beeswax, hard paraffin or cetyl alcohol. Sweetening agents such as those set forth above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water provide the active compound in admixture with a dispersing or wetting agent, suspending agent and one or more preservatives. Suitable dispersing or wetting agents and suspending agents are exemplified by those already mentioned above. Additional excipients, for example, sweetening, flavouring, and colouring agents may also be present. The pharmaceutical compositions of IL-21 or the IL-21 mimetic, optionally together with the combination agent for use in treating cancer according to the present invention may also be in the form of oil-in-water emulsions. The oily phase may be a vegetable oil, for example, olive oil or arachis oil, or a mineral oil, for example a liquid paraffin, or a mixture thereof. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of said partial esters with ethylene oxide, for example polyoxyethylene sorbitan monooleate. The emulsions may also contain sweetening and flavouring agents.

Syrups and elixirs may be formulated with sweetening agents, for example glycerol, propylene glycol, sorbitol or sucrose. Such formulations may also contain a demulcent, preservatives and flavouring and colouring agents. The pharmaceutical compositions may be in the form of a sterile injectable aqueous or oleaginous suspension. This suspension may be formulated according to the known methods using suitable dispersing or wetting agents and suspending agents described above. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution, and isotonic sodium chloride solution. In addition, sterile, fixed oils are conveniently employed as solvent or suspending medium. For this purpose, any bland fixed oil may be employed using synthetic mono- or diglycerides. In addition, fatty acids such as oleic acid find use in the preparation of injectables.

The compositions may also be in the form of suppositories for rectal administration of the compounds of the invention. These compositions can be prepared by mixing the drug with a suitable non-irritating excipient which is solid at ordinary temperatures but liquid at the rectal temperature and will thus melt in the rectum to release the drug. Such materials include cocoa butter and polyethylene glycols, for example.

For topical use, creams, ointments, jellies, solutions of suspensions, etc., containing the compounds of the invention are contemplated. For the purpose of this application, topical applications shall include mouth washes and gargles.

The IL-21 or the IL-21 mimetic, optionally together with the combination agent for use in treating cancer according to the present invention may also be administered in the form of liposome delivery systems, such as small unilamellar vesicles, large unilamellar vesicles, and multilamellar vesicles. Liposomes may be formed from a variety of phospholipids, such as cholesterol, stearylamine, or phosphatidylcholines.

In addition, some of the IL-21 or the IL-21 mimetic, optionally together with the combination agent for use in treating cancer according to the present invention may form solvates with water or common organic solvents. Such solvates are also encompassed within the scope of the invention.

If a solid carrier is used for oral administration, the preparation may be tabletted, placed in a hard gelatine capsule in powder or pellet form or it can be in the form of a troche or lozenge. The amount of solid carrier will vary widely but will usually be from about 25 mg to about 1 g. If a liquid carrier is used, the preparation may be in the form of a syrup, emulsion, soft gelatine capsule or sterile injectable liquid such as an aqueous or non-aqueous liquid suspension or solution.

The IL-21 or the IL-21 mimetic, optionally together with the combination agent for use in treating cancer according to the present invention may be administered to a mammal, especially a human, in need of such treatment. Such mammals include also animals, both domestic animals, e.g. household pets, and non-domestic animals such as wildlife. Pharmaceutical compositions containing a compound according to the invention may be administered one or more times per day or week, conveniently administered at mealtimes. An effective amount of such a pharmaceutical composition is the amount that provides a clinically significant effect. Such amounts will depend, in part, on the particular condition to be treated, age, weight, and general health of the patient, and other factors evident to those skilled in the art.

The invention provides in a particular embodiment the following:

Another object of the present invention is to provide a pharmaceutical formulation comprising IL-21, analogues or derivatives thereof, or optionally together with any other compound mentioned in the present application which is present in a concentration from 0.1 mg/ml to 100 mg/ml, and wherein said formulation has a pH from 2.0 to 10.0. The formulation may further comprise a buffer system, preservative(s), tonicity agent(s), chelating agent(s), stabilizers and surfactants. In one embodiment of the invention the pharmaceutical formulation is an aqueous formulation, i.e. formulation comprising water. Such formulation is typically a solution or a suspension. In a further embodiment of the invention the pharmaceutical formulation is an aqueous solution. The term "aqueous formulation" is defined as a formulation comprising at least 50 %w/w water. Likewise, the term "aqueous solution" is defined as a solution comprising at least 50 %w/w water, and the term "aqueous suspension" is defined as a suspension comprising at least 50 %w/w water.

In another embodiment the pharmaceutical formulation is a freeze-dried formulation, whereto the physician or the patient adds solvents and/or diluents prior to use.

In another embodiment the pharmaceutical formulation is a dried formulation (e.g. freeze-dried or spray-dried) ready for use without any prior dissolution.

In a further aspect the invention relates to a pharmaceutical formulation comprising an aqueous solution of IL-21 or any other compound as mentioned above and a buffer, wherein said compound is present in a concentration from 0.1 mg/ml or as mentioned above, preferably from 0.5 mg/ml - 50 mg/ml and wherein said formulation has a pH from about 2.0 to about 10.0. Preferred pH is from 3.0 to about 8.0. Particular preferred range is from 4.0-6.0, such as for example the ranges 4.0-4.5, 4.5 -5.0, 5.0-5.5 and 5.5-6.0.

In another embodiment of the invention the pH of the formulation is selected from the list consisting of 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, and 10.0.

In a further embodiment of the invention the buffer is selected from the group consisting of sodium acetate, sodium carbonate, citrate, glycylglycine, histidine, glycine, lysine, arginine, sodium dihydrogen phosphate, disodium hydrogen phosphate, sodium phosphate, and tris(hydroxymethyl)-aminomethan, bicine, tricine, malic acid, succinate, maleic acid, fumaric acid, tartaric acid, aspartic acid or mixtures thereof. Each one of these specific buffers constitutes an alternative embodiment of the invention.

In a further embodiment of the invention the formulation further comprises a pharmaceutically acceptable antimicrobial preservative. In a further embodiment of the invention the preservative is selected from the group consisting of phenol, o-cresol, m-cresol, p-cresol, methyl p-hydroxybenzoate, propyl p-hydroxybenzoate, 2-phenoxyethanol, butyl p-hydroxybenzoate, 2-phenylethanol, benzyl alcohol, chlorobutanol, and thiomersal, bronopol, benzoic acid, imidurea, chlorohexidine, sodium dehydroacetate, chlorocresol, ethyl p-hydroxybenzoate, benzethonium chloride, chlorphenesine (3p-chlorphenoxypropane-1,2-diol) or mixtures thereof. In a further embodiment of the invention the preservative is present in a concentration from 0.1 mg/ml to 20 mg/ml. In a further embodiment of the invention the preservative is present in a concentration from 0.1 mg/ml to 5 mg/ml. In a further embodiment of the invention the preservative is present in a concentration from 5 mg/ml to 10 mg/ml. In a further embodiment of the invention the preservative is present in a concentration from 10 mg/ml to 20 mg/ml. Each one of these specific preservatives constitutes an alternative embodiment of the invention. The use of a preservative in pharmaceutical compositions is well-known to the skilled person. For convenience reference is made to Remington: The Science and Practice of Pharmacy, 19th edition, 1995.

In a further embodiment of the invention the formulation further comprises an isotonic agent. In a further embodiment of the invention the isotonic agent is selected from the group consisting of a salt (e.g. sodium chloride), a sugar or sugar alcohol, an amino acid (e.g. L-glycine, L-histidine, arginine, lysine, isoleucine, aspartic add, tryptophan, threonine), an alditol (e.g. glycerol (glycerine), 1,2-propanediol (propyleneglycol), 1,3-propanediol, 1,3-butanediol) polyethyleneglycol (e.g. PEG400), or mixtures thereof. Any sugar such as mono-, di-, or polysaccharides, or water-soluble glucans, including for example fructose, glucose, mannose, sorbose, xylose, maltose, lactose, sucrose, trehalose, dextran, pullulan, dextrin, cyclodextrin, soluble starch, hydroxyethyl starch and carboxymethylcellulose-Na may be used. In one embodiment the sugar additive is sucrose. Sugar alcohol is defined as a C4-C8 hydrocarbon having at least one -OH group and includes, for example, mannitol, sorbitol, inositol, galactitol, dulcitol, xylitol, and arabitol. In one embodiment the sugar alcohol additive is mannitol. The sugars or sugar alcohols mentioned above may be used individually or in combination. There is no fixed limit to the amount used, as long as the sugar or sugar alcohol is soluble in the liquid preparation and does not adversely effect the stabilizing effects achieved using the methods of the invention. In one embodiment, the sugar or sugar alcohol concentration is between about 1 mg/ml and about 150 mg/ml. In a further embodiment of the invention the isotonic agent is present in a concentration from 1 mg/ml to 50 mg/ml. In a further embodiment of the invention the isotonic agent is present in a concentration from 1 mg/ml to 7 mg/ml. In a further embodiment of the invention the isotonic agent is present in a concentration from 8 mg/ml to 24 mg/ml. In a further embodiment of the invention the isotonic agent is present in a concentration from 25 mg/ml to 50 mg/ml. Each one of these specific isotonic agents constitutes an alternative embodiment of the invention. The use of an isotonic agent in pharmaceutical compositions is well-known to the skilled person. For convenience reference is made to Remington: The Science and Practice of Pharmacy, 19th edition, 1995.

In a further embodiment of the invention the formulation further comprises a chelating agent. In a further embodiment of the invention the chelating agent is selected from salts of ethylenediaminetetraacetic acid (EDTA), citric acid, and aspartic acid, and mixtures thereof. In a further embodiment of the invention the chelating agent is present in a concentration from 0.1mg/ml to 5mg/ml. In a further embodiment of the invention the chelating agent is present in a concentration from 0.1mg/ml to 2mg/ml. In a further embodiment of the invention the chelating agent is present in a concentration from 2mg/ml to 5mg/ml. Each one of these specific chelating agents constitutes an alternative embodiment of the invention. The use of a chelating agent in pharmaceutical compositions is well-known to the skilled person. For convenience reference is made to Remington: The Science and Practice of Pharmacy, 19th edition, 1995.

In a further embodiment of the invention the formulation further comprises a stabilizer. The use of a stabilizer in pharmaceutical compositions is well-known to the skilled person. For convenience reference is made to Remington: The Science and Practice of Pharmacy, 19th edition, 1995.

More particularly, compositions of the invention are stabilized liquid pharmaceutical compositions whose therapeutically active components include a polypeptide that possibly exhibits aggregate formation during storage in liquid pharmaceutical formulations. By "aggregate formation" is intended a physical interaction between the polypeptide molecules that results in formation of oligomers, which may remain soluble, or large visible aggregates that precipitate from the solution. By "during storage" is intended a liquid pharmaceutical composition or formulation once prepared, is not immediately administered to a subject. Rather, following preparation, it is packaged for storage, either in a liquid form, in a frozen state, or in a dried form for later reconstitution into a liquid form or other form suitable for administration to a subject. By "dried form" is intended the liquid pharmaceutical composition or formulation is dried either by freeze drying (i.e., lyophilization; see, for example, Williams and Polli (1984) J. Parenteral Sci. Technol. 38:48-59), spray drying (see Masters (1991) in Spray-Drying Handbook (5th ed; Longman Scientific and Technical, Essez, U.K.), pp. 491-676; Broadhead et al. (1992) Drug Devel. Ind. Pharm. 18:1169-1206; and Mumenthaler et al. (1994) Pharm. Res. 11:12-20), or air drying (Carpenter and Crowe (1988) Cryobiology 25:459-470; and Roser (1991) Biopharm. 4:47-53). Aggregate formation by a polypeptide during storage of a liquid pharmaceutical composition can adversely affect biological activity of that polypeptide, resulting in loss of therapeutic efficacy of the pharmaceutical composition. Furthermore, aggregate formation may cause other problems such as blockage of tubing, membranes, or pumps when the polypeptide-containing pharmaceutical composition is administered using an infusion system.

The pharmaceutical compositions of the invention may further comprise an amount of an amino acid base sufficient to decrease aggregate formation by the polypeptide during storage of the composition. By "amino acid base" is intended an amino acid or a combination of amino acids, where any given amino acid is present either in its free base form or in its salt form. Where a combination of amino acids is used, all of the amino acids may be present in their free base forms, all may be present in their salt forms, or some may be present in their free base forms while others are present in their salt forms. In one embodiment, amino acids to use in preparing the compositions of the invention are those carrying a charged side chain, such as arginine, lysine, aspartic acid, and glutamic acid. Any stereoisomer (i.e., L, D, or DL isomer) of a particular amino acid (e.g. glycine, methionine, histidine, imidazole, arginine, lysine, isoleucine, aspartic acid, tryptophan, threonine and mixtures thereof) or combinations of these stereoisomers, may be present in the pharmaceutical compositions of the invention so long as the particular amino acid is present either in its free base form or its salt form. In one embodiment the L-stereoisomer is used. Compositions of the invention may also be formulated with analogues of these amino acids. By "amino acid analogue" is intended a derivative of the naturally occurring amino acid that brings about the desired effect of decreasing aggregate formation by the polypeptide during storage of the liquid pharmaceutical compositions of the invention. Suitable arginine analogues include, for example, aminoguanidine, ornithine and N-monoethyl L-arginine, suitable methionine analogues include ethionine and buthionine and suitable cysteine analogues include S-methyl-L cysteine. As with the other amino acids, the amino acid analogues are incorporated into the compositions in either their free base form or their salt form. In a further embodiment of the invention the amino acids or amino acid analogues are used in a concentration, which is sufficient to prevent or delay aggregation of the protein.

In a further embodiment of the invention methionine (or other sulphuric amino acids or amino acid analogous) may be added to inhibit oxidation of methionine residues to methionine sulfoxide when the polypeptide acting as the therapeutic agent is a polypeptide comprising at least one methionine residue susceptible to such oxidation. By "inhibit" is intended minimal accumulation of methionine oxidized species over time. Inhibiting methionine oxidation results in greater retention of the polypeptide in its proper molecular form. Any stereoisomer of methionine (L, D, or DL isomer) or combinations thereof can be used. The amount to be added should be an amount sufficient to inhibit oxidation of the methionine residues such that the amount of methionine sulfoxide is acceptable to regulatory agencies. Typically, this means that the composition contains no more than about 10% to about 30% methionine sulfoxide. Generally, this can be achieved by adding methionine such that the ratio of methionine added to methionine residues ranges from about 1:1 to about 1000:1, such as 10:1 1 to about 100:1.

In a further embodiment of the invention the formulation further comprises a stabilizer selected from the group of high molecular weight polymers or low molecular compounds. In a further embodiment of the invention the stabilizer is selected from polyethylene glycol (e.g. PEG 3350), polyvinyl alcohol (PVA), polyvinylpyrrolidone, carboxy-/hydroxycellulose or derivates thereof (e.g. HPC, HPC-SL, HPC-L and HPMC), cyclodextrins, sulphur-containing substances as monothioglycerol, thioglycolic acid and 2-methylthioethanol, and different salts (e.g. sodium chloride). Each one of these specific stabilizers constitutes an alternative embodiment of the invention.

The pharmaceutical compositions may also comprise additional stabilizing agents, which further enhance stability of a therapeutically active polypeptide therein. Stabilizing agents of particular interest to the present invention include, but are not limited to, methionine and EDTA, which protect the polypeptide against methionine oxidation, and a nonionic surfactant, which protects the polypeptide against aggregation associated with freeze-thawing or mechanical shearing.

In a further embodiment of the invention the formulation further comprises a surfactant. In a further embodiment of the invention the surfactant is selected from a detergent, ethoxylated castor oil, polyglycolyzed glycerides, acetylated monoglycerides,

sorbitan fatty acid esters, polyoxypropylene-polyoxyethylene block polymers (eg. poloxamers such as Pluronic^{®} F68, poloxamer 188 and 407, Triton X-100), polyoxyethylene sorbitan fatty acid esters, polyoxyethylene and polyethylene derivatives such as alkylated and alkoxylated derivatives (tweens, e.g. Tween-20, Tween-40, Tween-80 and Brij-35), monoglycerides or ethoxylated derivatives thereof, diglycerides or polyoxyethylene derivatives thereof, alcohols, glycerol, lectins and phospholipids (eg. phosphatidyl serine, phosphatidyl choline, phosphatidyl ethanolamine, phosphatidyl inositol, diphosphatidyl glycerol and sphingomyelin), derivates of phospholipids (eg. dipalmitoyl phosphatidic acid) and lysophospholipids (eg. palmitoyl lysophosphatidyl-L-serine and 1-acyl-sn-glycero-3-phosphate esters of ethanolamine, choline, serine or threonine) and alkyl, alkoxyl (alkyl ester), alkoxy (alkyl ether)- derivatives of lysophosphatidyl and phosphatidylcholines, e.g. lauroyl and myristoyl derivatives of lysophosphatidylcholine, dipalmitoylphosphatidylcholine, and modifications of the polar head group, that is cholines, ethanolamines, phosphatidic acid, serines, threonines, glycerol, inositol, and the positively charged DODAC, DOTMA, DCP, BISHOP, lysophosphatidylserine and lysophosphatidylthreonine, and glycerophospholipids (eg. cephalins), glyceroglycolipids (eg. galactopyransoide), sphingoglycolipids (eg. ceramides, gangliosides), dodecylphosphocholine, hen egg lysolecithin, fusidic acid derivatives- (e.g. sodium tauro-dihydrofusidate etc.), long-chain fatty acids and salts thereof C6-C12 (eg. oleic acid and caprylic acid), acylcarnitines and derivatives, N^{α}-acylated derivatives of lysine, arginine or histidine, or side-chain acylated derivatives of lysine or arginine, N^{α}-acylated derivatives of dipeptides comprising any combination of lysine, arginine or histidine and a neutral or acidic amino acid, N^{α}-acylated derivative of a tripeptide comprising any combination of a neutral amino acid and two charged amino acids, DSS (docusate sodium, CAS registry no [577-11-7]), docusate calcium, CAS registry no [128-49-4]), docusate potassium, CAS registry no [7491-09-0]), SDS (sodium dodecyl sulphate or sodium lauryl sulphate), sodium caprylate, cholic acid or derivatives thereof, bile acids and salts thereof and glycine or taurine conjugates, ursodeoxycholic acid, sodium cholate, sodium deoxycholate, sodium taurocholate, sodium glycocholate, N-Hexadecyl-N,N-dimethyl-3-ammonio-1-propanesulfonate, anionic (alkyl-aryl-sulphonates) monovalent surfactants, zwitterionic surfactants (e.g. N-alkyl-N,N-dimethylammonio-1-propanesulfonates, 3-chofamido-1-propyldimethylammonio-1-propanesulfonate, cationic surfactants (quaternary ammonium bases) (e.g. cetyl-trimethylammonium bromide, cetylpyridinium chloride), nonionic surfactants (eg. Dodecyl- β-D-glucopyranoside), poloxamines (eg. Tetronic's), which are tetrafunctional block copolymers derived from sequential addition of propylene oxide and ethylene oxide to ethylenediamine, or the surfactant may be selected from the group of imidazoline derivatives, or mixtures thereof. Each one of these specific surfactants constitutes an alternative embodiment of the invention.

The use of a surfactant in pharmaceutical compositions is well-known to the skilled person. For convenience reference is made to Remington: The Science and Practice of Pharmacy, 19th edition, 1995.

It is possible that other ingredients may be present in the peptide pharmaceutical formulation of the present invention. Such additional ingredients may include wetting agents, emulsifiers, antioxidants, bulking agents, tonicity modifiers, chelating agents, metal ions, oleaginous vehicles, proteins (e.g., human serum albumin, gelatine or proteins) and a zwitterion (e.g., an amino acid such as betaine, taurine, arginine, glycine, lysine and histidine). Such additional ingredients, of course, should not adversely affect the overall stability of the pharmaceutical formulation of the present invention.

Pharmaceutical compositions containing IL-21 or any other compound as mentioned above according to the present invention may be administered to a patient in need of such treatment at several sites, for example, at topical sites, for example, skin and mucosal sites, at sites which bypass absorption, for example, administration in an artery, in a vein, in the heart, and at sites which involve absorption, for example, administration in the skin, under the skin, in a muscle or in the abdomen.

Administration of pharmaceutical compositions according to the invention may be through several routes of administration, for example, lingual, sublingual, buccal, in the mouth, oral, in the stomach and intestine, nasal, pulmonary, for example, through the bronchioles and alveoli or a combination thereof, epidermal, dermal, transdermal, vaginal, rectal, ocular, for examples through the conjunctiva, uretal, and parenteral to patients in need of such a treatment.

Compositions of the current invention may be administered in several dosage forms, for example, as solutions, suspensions, emulsions, microemulsions, multiple emulsion, foams, salves, pastes, plasters, ointments, tablets, coated tablets, rinses, capsules, for example, hard gelatine capsules and soft gelatine capsules, suppositories, rectal capsules, drops, gels, sprays, powder, aerosols, inhalants, eye drops, ophthalmic ointments, ophthalmic rinses, vaginal pessaries, vaginal rings, vaginal ointments, injection solution, in situ transforming solutions, for example in situ gelling, in situ setting, in situ precipitating, in situ crystallization, infusion solution, and implants.

Compositions of the invention may further be compounded in, or attached to, for example through covalent, hydrophobic and electrostatic interactions, a drug carrier, drug delivery system and advanced drug delivery system in order to further enhance stability of of IL-21 or any other compound as mentioned above, increase bioavailability, increase solubility, decrease adverse effects, achieve chronotherapy well known to those skilled in the art, and increase patient compliance or any combination thereof. Examples of carriers, drug delivery systems and advanced drug delivery systems include, but are not limited to, polymers, for example cellulose and derivatives, polysaccharides, for example dextran and derivatives, starch and derivatives, poly(vinyl alcohol), acrylate and methacrylate polymers, polylactic and polyglycolic acid and block co-polymers thereof, polyethylene glycols, carrier proteins, for example albumin, gels, for example, thermogelling systems, for example block co-polymeric systems well known to those skilled in the art, micelles, liposomes, microspheres, nanoparticulates, liquid crystals and dispersions thereof, L2 phase and dispersions there of, well known to those skilled in the art of phase behaviour in lipid-water systems, polymeric micelles, multiple emulsions, self-emulsifying, self-microemulsifying, cyclodextrins and derivatives thereof, and dendrimers.

Compositions of the current invention are useful in the formulation of solids, semisolids, powder and solutions for pulmonary administration of IL-21 or any other compound as mentioned above using, for example a metered dose inhaler, dry powder inhaler and a nebulizer, all being devices well known to those skilled in the art.

Compositions of the current invention are specifically useful in the formulation of controlled, sustained, protracting, retarded, and slow release drug delivery systems. More specifically, but not limited to, compositions are useful in formulation of parenteral controlled release and sustained release systems (both systems leading to a many-fold reduction in number of administrations), well known to those skilled in the art. Even more preferably, are controlled release and sustained release systems administered subcutaneous. Without limiting the scope of the invention, examples of useful controlled release system and compositions are hydrogels, oleaginous gels, liquid crystals, polymeric micelles, microspheres, nanoparticles,

Methods to produce controlled release systems useful for compositions of the current invention include, but are not limited to, crystallization, condensation, co-crystallization, precipitation, co-precipitation, emulsification, dispersion, high pressure homogenisation, encapsulation, spray drying, microencapsulating, coacervation, phase separation, solvent evaporation to produce microspheres, extrusion and supercritical fluid processes. General reference is made to Handbook of Pharmaceutical Controlled Release (Wise, D.L., ed. Marcel Dekker, New York, 2000) and Drug and the Pharmaceutical Sciences vol. 99: Protein Formulation and Delivery (MacNally, E.J., ed. Marcel Dekker, New York, 2000).

Parenteral administration may be performed by subcutaneous, intramuscular, intraperitoneal or intravenous injection by means of a syringe, optionally a pen-like syringe. Alternatively, parenteral administration can be performed by means of an infusion pump. A further option is a composition which may be a solution or suspension for the administration of IL-21 or any other compound as mentioned above, in the form of a nasal or pulmonal spray. As a still further option, the pharmaceutical compositions containing IL-21 or any other compound as mentioned above can also be adapted to transdermal administration, e.g. by needle-free injection or from a patch, optionally an iontophoretic patch, or transmucosal, e.g. buccal, administration.

The term "stabilized formulation" refers to a formulation with increased physical stability, increased chemical stability or increased physical and chemical stability.

The term "physical stability" of the protein formulation as used herein refers to the tendency of the protein to form biologically inactive and/or insoluble aggregates of the protein as a result of exposure of the protein to thermo-mechanical stresses and/or interaction with interfaces and surfaces that are destabilizing, such as hydrophobic surfaces and interfaces. Physical stability of the aqueous protein formulations is evaluated by means of visual inspection and/or turbidity measurements after exposing the formulation filled in suitable containers (e.g. cartridges or vials) to mechanical/physical stress (e.g. agitation) at different temperatures for various time periods. Visual inspection of the formulations is performed in a sharp focused light with a dark background. The turbidity of the formulation is characterized by a visual score ranking the degree of turbidity for instance on a scale from 0 to 3 (a formulation showing no turbidity corresponds to a visual score 0, and a formulation showing visual turbidity in daylight corresponds to visual score 3). A formulation is classified physically unstable with respect to protein aggregation, when it shows visual turbidity in daylight. Alternatively, the turbidity of the formulation can be evaluated by simple turbidity measurements well-known to the skilled person. Physical stability of the aqueous protein formulations can also be evaluated by using a spectroscopic agent or probe of the conformational status of the protein. The probe is preferably a small molecule that preferentially binds to a non-native conformer of the protein. One example of a small molecular spectroscopic probe of protein structure is Thioflavin T. Thioflavin T is a fluorescent dye that has been widely used for the detection of amyloid fibrils. In the presence of fibrils, and perhaps other protein configurations as well, Thioflavin T gives rise to a new excitation maximum at about 450 nm and enhanced emission at about 482 nm when bound to a fibril protein form. Unbound Thioflavin T is essentially non-fluorescent at the wavelengths.

Other small molecules can be used as probes of the changes in protein structure from native to non-native states. For instance the "hydrophobic patch" probes that bind preferentially to exposed hydrophobic patches of a protein. The hydrophobic patches are generally buried within the tertiary structure of a protein in its native state, but become exposed as a protein begins to unfold or denature. Examples of these small molecular, spectroscopic probes are aromatic, hydrophobic dyes, such as antrhacene, acridine, phenanthroline or the like. Other spectroscopic probes are metal-amino acid complexes, such as cobalt metal complexes of hydrophobic amino acids, such as phenylalanine, leucine, isoleucine, methionine, and valine, or the like..

The term "chemical stability" of the protein formulation as used herein refers to chemical covalent changes in the protein structure leading to formation of chemical degradation products with potential less biological potency and/or potential increased immunogenic properties compared to the native protein structure. Various chemical degradation products can be formed depending on the type and nature of the native protein and the environment to which the protein is exposed. Elimination of chemical degradation can most probably not be completely avoided and increasing amounts of chemical degradation products is often seen during storage and use of the protein formulation as well-known by the person skilled in the art. Most proteins are prone to deamidation, a process in which the side chain amide group in glutaminyl or asparaginyl residues is hydrolysed to form a free carboxylic acid. Other degradation pathways involves formation of high molecular weight transformation products where two or more protein molecules are covalently bound to each other through transamidation and/or disulfide interactions leading to formation of covalently bound dimer, oligomer and polymer degradation products (Stability of Protein Pharmaceuticals, Ahern. T.J. & Manning M.C., Plenum Press, New York 1992). Oxidation (of for instance methionine residues) can be mentioned as another variant of chemical degradation. The chemical stability of the protein formulation can be evaluated by measuring the amount of the chemical degradation products at various time-points after exposure to different environmental conditions (the formation of degradation products can often be accelerated by for instance increasing temperature). The amount of each individual degradation product is often determined by separation of the degradation products depending on molecule size and/or charge using various chromatography techniques (e.g. SEC-HPLC and/or RP-HPLC).

Hence, as outlined above, a "stabilized formulation" refers to a formulation with increased physical stability, increased chemical stability or increased physical and chemical stability. In general, a formulation must be stable during use and storage (in compliance with recommended use and storage conditions) until the expiration date is reached.

In one embodiment of the invention the pharmaceutical formulation comprising IL-21 or any other compound as mentioned above is stable for more than 6 weeks of usage and for more than 3 years of storage.

In another embodiment of the invention the pharmaceutical formulation comprising IL-21 or any other compound as mentioned above is stable for more than 4 weeks of usage and for more than 3 years of storage.

In a further embodiment of the invention the pharmaceutical formulation comprising IL-21 or any other compound as mentioned above is stable for more than 4 weeks of usage and for more than two years of storage.

In an even further embodiment of the invention the pharmaceutical formulation comprising IL-21 or any other compound as mentioned above is stable for more than 2 weeks of usage and for more than two years of storage.

In an even further embodiment of the invention the pharmaceutical formulation comprising IL-21 or any other compound as mentioned above is stable for more than 1 weeks of usage and for more than 18 months of storage.

In an even further embodiment of the invention the pharmaceutical formulation comprising IL-21 or any other compound as mentioned above is stable for one day of usage and for more than 18 months of storage.

### EXAMPLES

### PHARMACOLOGICAL METHODS

The following in vitro method is used to investigate enhancement of ADCC:

Target cells expressing the target antigen are incubated with the antibody against the target antigen and peripheral blood mononuclear cells, NK cells, neutrophils, macrophages, monocytes or DC as effector cells. Effector cells may be pre-incubated for 1 to 10 days with IL-21, or IL-21 may be added to the culture containing both effector and target cells. Other compounds that can enhance ADCC might be included in the culture or pre-incubation culture. Efficiency of ADCC will be measured as specific ⁵¹Cr release from the target cells or as LDH activity as described previously (Golay et al., Haematologica 88:1002-1012, 2003 or Liu et al., Cancer Immun 2:13, 2002 or Watanabe et al., Breast Cancer Res Treat 53:199-207, 1999)

Determination of ADCC using a flow cytometry based assay as described previously (Flieger et. al., J Immunother 23:480-486, 2000 or Flieger et al., J Immunol Methods 180:1-13, 1995 or Flieger et al., Hybridoma 18:63-68, 1999)

Determination of ADCP through two-color fluorescence assay as described in Watanabe et al., Breast Cancer Res Treat 53:199-207, 1999 or Akewanlop et al., Cancer Res 61:4061-4065,2001

An in vivo method for determining the enhancement of ADCC is outlined below:

Leukaemia cells or transformed cells are injected i.v., i.p. or s.c. in syngeneic animals followed by treatment with the therapeutic antibody recognising an antigen expressed by the leukaemia cells or transformed cells, with or without IL-21 therapy. Endpoints are tumor burden and survival. The involvement of ADCC may be confirmed by the use of FcγRI blocking antibodies or by the use of FcγRI-deficient mice.

An in vivo method to investigate enhancement of ADCC towards target cells of human origin is described previously in Zhang et al., Blood 102:284-288, 2003 or Flavell et al. Cancer Res 58:5787-5794, 1998. According to these models human leukaemia cells or transformed cells are injected i.v., i.p. or s.c. in SCID mice followed by treatment with the therapeutic antibody recognising an antigen expressed by the leukaemia cells or transformed cells, with or without IL-21 therapy.

Tumor cell lines, e.g. Lewis Lung Carcinoma (LLC) cells or B16-F10 melanoma cells or renca renal cell carcinoma cells or 4T1 breast carcinoma cells are implanted s.c. in syngeneic mice. When the tumors become palpable, the mice are treated with IL-21 in combination with other anti-cancer agents as described in this application. The methodology is described in Palumbo et al. , Cancer Res. 62:6966-6972 (2002); Bove et al., Biochem Biophys Res Commun 291:1001-1005 (2002); Wigginton et al., J Immunol 169:4467-4474 (2002).

Tumor cell lines, e.g. Lewis Lung Carcinoma (LLC) cells or B16-F10 melanoma cells are implanted s.c. in syngeneic mice. The primary tumor is removed after 1-4 weeks, and the mice are treated with IL-21 in combination with other anti-cancer agents as described in this application. The methodology is described in Palumbo et al., Cancer Res. 62:6966-6972 (2002);

Tumor cell lines, e.g. Lewis Lung Carcinoma (LLC) cells or B16-F10 melanoma cells or renca renal cell carcinoma cells are injected i.v. in syngeneic mice and the mice are treated with IL-21 in combination with other anti-cancer agents as described in this application. The methodology is described in Amirkhosravi et al., Thromb.Haemost. 87:930-936 (2002);Hosaka et al., Cancer Lett 161:231-240 (2000); Maini et al., In vivo 17:119-123 (2003)

Renca renal cell carcinoma cells are injected intra-renally in one kidney in syngeneic mice. The primary tumor is removed after 1-4 weeks, and the mice are treated with IL-21 in combination with other anti-cancer agents as described in this application. The methodology is described in Murphy et al., J Immunol 170:2727-2733 (2003)
1. A combination of IL-21, analogues or derivatives thereof, and one or more of the following:
   I. Agents that induce tumor cell death or death of virus-infected cells:
      a) conventional chemotherapy
      b) radiation therapy
      c) monoclonal antibodies
      d) cell cycle control/apoptosis regulators
      e) growth factor and signal transduction modulators
      f) inhibitors of tumor vascularisation (angiogenesis inhibitors, antiangiogenesis drugs)
      g) viral targeting (the use of a recombinant virus to destroy tumor cells)
      h) anti-viral agents
      i) hormonal agents
         or
   II. Agents that enhance the immune response against tumor cells or virus-infected cells:
      j) immune system activators
      k) immune system inhibitors (e.g. agents that inhibit immune signals downregulating the immune response), including anti-anergic agents
      I) therapeutic vaccines
         or
   III. Agents that interfere with tumor growth, metastasis or spread of virus-infected cells:
      m) integrins, cell adhesion molecules modulators
      n) anti-metastatics
      o) endothelial cell modulators
         or
   IV. Internal vaccination.
   V. Tissue factor antagonist and other factors influencing the coagulation cascade
      p) anti Factor Xa, anti Factor IIa inhibitors, anti-fibrinogenic agents
      q) pentasaccharides etc.
   VI. Immunosuppressive / immunomodulatory agents
      r) agents with influence on T-lymphocyte homing e.g. FTY-720
      s) calcineurin inhibitors
      t) TOR inhibitors.
2. A combination according to clause 1 comprising human IL-21.
3. A combination according to clause 1 comprising an analogue of IL-21.
4. A combination according to clause 1 comprising a derivative of IL-21.
5. A combination according to any of the clauses 1-4, wherein the combination comprises dacarbazine (DTIC).
6. A combination according to any of the clauses 1-4, wherein the combination comprises radiation therapy.
7. A combination according to any of the clauses 1-4, wherein the combination comprises antibodies such as Rituximab, Alemtuzumab, Trastuzumab, Gemtuzumab, Gemtuzumabozogamicin (Myelotarg ®, Wyeth) Cetuximab (Erbitux™), Bevacizumab, HuMax-CD20, Hu-Max-EGFr, Zamyl, Pertuzumab, antibodies against tissue factor, killer Ig-like receptors (KIR) and laminin-5.
8. A combination according to clause 7 wherein the combination comprises Rituximab.
9. A combination according to clause 7, wherein the combination comprises Cetuximab.
10. A combination according to clause 7, wherein the therapeutic antibody is further combined with additional ADCC-enhancing compounds, ex. blocking anti-KIR antibodies or activating NKG2A antibodies or IL-2.
11. A combination according to any of the clauses 1-4, wherein the combination comprises antibodies against viral antigens.
12. A combination according to any of the clauses 1-4, wherein the combination comprises one or more cell-cycle regulators and/or apoptosis-inducing agents.
13. A combination according to clause 12 wherein the combination comprises compounds selected from the group comprising cdc-25, NSC 663284, flavopiridol, 7-hydroxystaurosporine, roscovitine , BIBR1532 SOT-095, TNF-related apoptosis-inducing ligand (TRAIL)/apoptosis-2 ligand (Apo-2L), antibodies that activate TRAIL receptors, IFNα and anti-sense Bcl-2.
14. A combination according to any of the clauses 1-4, wherein the combination comprises growth factor inhibitors.
15. A combination according to clause 14, wherein the growth factor inhibitors are selected from the group comprising Herceptin (monoclonal antibody), cetuximab (monoclonal antibody), Tarceva (low molecular weight inhibitor), and Iressa (low molecular weight inhibitor).
16. A combination according to clauses 14-15 wherein the combination comprises Herceptin.
17. A combination according to any of the clauses 1-4, wherein the combination comprises anti-angiogenesis drug.
18. A combination according to clause 17, wherein the anti-angiogenesis drug is selected from the group comprising: avastin, neovastat, thalidomide, PTK787, ZK222584, ZD-6474, SU6668, PD547,632, VEGF-Trap, CEP-7055, NM-3, SU11248.
19. A combination according to any of the clauses 1-4, wherein the combination comprises viral targeting.
20. A combination according to any of the clauses 1-4, wherein the combination comprises hormone therapy.
21. A combination according to any of the clauses 1-4, wherein the combination comprises one or more adjuvants.
22. A combination according to clause 21, wherein the adjuvants are selected from the group comprising: QS21, GM-CSF and CpG oligodeoxynucleotides, lipopolysaccharide and polyinosinic:polycytidylic acid.
23. A combination according to any of the clauses 1-4, wherein the combination comprises one or more cytokines.
24. A combination according to clause 23 wherein the cytokines is one or more of the compounds selected from the group comprising: IFN-α, IFN-β, IFN-γ, IL-2, PEG-IL-2, IL-4, IL-6, IL-7, IL-12, IL-13, IL-15, IL-18, IL-23, IL-27, IL-28a, IL-28b, IL-29, GM-CSF, Flt3 ligand or stem cell factor.
25. A combination according to clauses 23 - 24 wherein the cytokines is one or more of the compounds selected from the group comprising IFN-α, IFN-β, IFN-γ, PEG-IL-2, IL-18, IL-23, IL-27, IL-28a, IL-28b, IL-29.
26. A combination according to any of the clauses 1-4 and 23 wherein the combination comprises one or more of the compounds selected from the group comprising: IL-2, PEG-IL-2, IL-7, IL-12, IL-15, and IFN-α.
27. A combination according to any of the clauses 14 and 23 - 24 wherein the combination comprises IL-12.
28. A combination according to any of the clauses 1-4 and 23-26 wherein the combination comprises IFN-α.
29. A combination according to any of the clauses 1-4 and 23-26, wherein the combination comprises PEG-IL-2.
30. A combination according to any of the clauses 1-4 and 23 wherein the combination comprises at least two IL-2, PEG-IL-2, IL-7, IL-12, IL-15, and IFN-α.
31. A combination according to any of the clauses 1-4 and 23 - 24 wherein the combination comprises at least one of the following: IL-2, PEG-IL-2, IL-7, IL-12, IL-15, and IFN-α and one additional active component.
32. A combination according to any of the clauses 1-4 and 23 - 24 wherein the combination comprises at least one of the following: IL-2, PEG-IL-2, IL-7, IL-12, IL-15, and IFN-α and one additional cytokine.
33. A combination according to any of the clauses 1-4 and 23 - 24 wherein the combination comprises IFN-α and GM-CSF.
34. A combination according to any of the clauses 1-4 and 23 wherein the combination comprises IFN-α and thymopentin.
35. A combination according to any of the clauses 1-4 and 23 wherein the combination comprises IFN-γ.
36. A combination according to any of the clauses 1-4 and 23 wherein the combination comprises autologous TILs and IFN-α.
37. A combination according to any of the clauses 1-4 and 23 wherein the combination comprises IFN-α and IL-12.
38. A combination according to any of the clauses 1-4 and 23 wherein the combination comprises Cis-platin and IFN-α.
39. A combination according to any of the clauses 1-4 and 23 wherein the combination comprises Cis-platin, tamoxifen and IFN-α.
40. A combination according to any of the clauses 1 -4 and 23 wherein the combination comprises Cis-platin, DTIC, tamoxifen and IFN-α.
41. A combination according to any of the clauses 1 -4 and 23 wherein the combination comprises Cis-platin, DTIC, tamoxifen and GM-CSF.
42. A combination according to any of the clauses 1-4 and 23 wherein the combination comprises Cis-platin, Carmustine, DTIC and IFN-α.
43. A combination according to any of the clauses 1-4 and 23 wherein the combination comprises Cis-platin, Carmustine, DTIC, tamoxifen and IFN-α.
44. A combination according to any of the clauses 1-4 and 23 wherein the combination comprises Cis-platin, Carmustine, DTIC, carboplatin, tamoxifen and IFN-α.
45. A combination according to any of the clauses 1-4 and 23 wherein the combination comprises Cis-platin, DTIC, Vinblastine, tamoxifen and IFN-α.
46. A combination according to any of the clauses 1-4 and 23 wherein the combination comprises Cis-platin, Vinblastine, temozolomide and IFN-α.
47. A combination according to any of the clauses 1-4 and 23 wherein the combination comprises Cis-platin, Carmustine, DTIC, Vindesine, tamoxifen and IFN-α.
48. A combination according to any of the clauses 1-4 and 23 wherein the combination comprisescis-platin, tamoxifen and IFN-α.
49. A combination according to any of the clauses 1-4 and 23 wherein the combination comprises Cis-platin, Vinblastine, DTIC and IFN-α.
50. A combination according to any of the clauses 1 -4 and 23 wherein the combination comprises DTIC and IFN-α.
51. A combination according to any of the clauses 1-4 and 23 wherein the combination comprises DTIC, GM-CSF and IFN-α.
52. A combination according to any of the clauses 1-4 and 23 wherein the combination comprises DTIC, thymosin-a and IFN-α.
53. A combination according to any of the clauses 1-4 and 23 wherein the combination comprises Vinblastine and IFN-α.
54. A combination according to any of the clauses 1-4 and 23 wherein the combination comprises 5-fluorouracil and IFN-α.
55. A combination according to any of the clauses 1 -4 and 26-27 wherein the combination comprises Fotemustine and IFN-α.
56. A combination according to any of the clauses 1-4 and 23 wherein the combination comprises Gemcitabine and IFN-α.
57. A combination according to clauses wherein the combinations further comprises IL-2.
58. A combination according to clauses 6-10 wherein the combination comprises passive immunotherapy.
59. A combination according to clauses 6-10 wherein the combination comprises cell adoptive therapy.
60. A combination according to clause 59, wherein the cell adoptive therapy is CD4+ or CD8+. T cells recognizing tumor specific antigens or tumor-associated antigens.
61. A combination according to clause 59, wherein the cell adoptive therapy is B cell expressing antibodies specific for tumor specific antigens or tumor-associated antigens.
62. A combination according to clause 59, wherein the cell adoptive therapy is NK cells that are able to kill the tumor cells.
63. A combination according to clause 59, wherein the cell adoptive therapy is dendritic cells (DC).
64. A combination according to clause 63, the dendritic cells are cultured in vivo with a DC expanding agent (e.g. GM-CSF or Flt3-L), loaded with tumor specific antigens or tumor associated antigens and reintroduced in vivo.
65. A combination according to any of the clauses 1-4 wherein the combination comprises one or more agents that break the tolerance to cancer, tumor or viral antigens.
66. A combination according to clause 65 wherein the agent MDX-010.
67. A combination according to clause 65 wherein the agents are antibodies against CTLA-4.
68. A combination according to any of the clauses 1-4 wherein the combination comprises one or more therapeutic vaccines with or without adjuvants, cytokines, CpG oligodeoxynucleotides, dendritic cells, GM-CSF, or heat-shock proteins.
69. A combination according to any of the clauses 1-4 wherein the combination comprises one or more an-metastatic agents, such as metalloproteinase inhibitors.
70. A combination according to any of the clauses 1-4 wherein the combination comprises internal vaccination therapy.
71. The use of a combination according to any of clauses 1-70 for the manufacture of a medicament for the treatment of cancer or viral infections.
72. A pharmaceutical composition comprising a combination according to any of the clauses 1-70 together with pharmaceutical acceptable additives.
73. A method of treating cancer or viral infection in a subject in need thereof by administrating an effective amount of a combination according to any of clauses 1-70.

## Claims

1. An antibody and an IL-21 polypeptide for use in treating cancer, wherein said antibody is selected from:
(i) HuMax-CD20, alemtuzumab, and Zamyl;
(ii) an antibody against tissue factor, laminin-5, EGF receptor, VEGF receptor, PDGF receptor, HER-2/neu, prostate-specific antigen (PSA), prostate stem cell antigen (PSCA), carcinoembryonic antigen (CEA), cancer antigen 125 (CA125), tumor-associated calcium signal transducer antigen (KSA), or VEGF;
(iii) an antibody that activates a TRAIL receptor; or
(iii) a monoclonal antibody against CTLA-4, leukocyte immunoglobulin-like receptor 1 (LIR), CD94, or NKG2A.

2. An antibody and an IL-21 polypeptide for use according to Claim 1, wherein the cancer is selected from the group consisting of metastatic malignant melanoma, renal cell carcinoma, ovarian cancer, small-cell lung cancer, non-small-cell lung cancer, breast cancer, colorectal cancer, prostate cancer, pancreatic cancer, bladder cancer, esophageal cancer, cervical cancer, endometrial cancer, lymphoma, and leukemia.

3. An antibody and an IL-21 polypeptide for use according to Claim 1 or Claim 2, wherein the IL-21 polypeptide is administered prior to the antibody.

4. An antibody and an IL-21 polypeptide for use according to Claim 1 or Claim 2, wherein the IL-21 polypeptide is administered simultaneously with the antibody.

5. An antibody and an IL-21 polypeptide for use according to Claim 1 or Claim 2, wherein the antibody is administered prior to the IL-21 polypeptide.

6. An antibody and an IL-21 polypeptide for use according to any previous claim, wherein the antibody is HuMax-CD20.

7. An antibody and an IL-21 polypeptide for use according to any of Claims 1-5, wherein said anti-VEGF antibody is bevacizumab (avastin), wherein said anti-EGF-R antibody is HuMax-EGFr, wherein said anti-Her2/neu antibody is pertuzumab, or wherein said anti-CTLA-4 antibody is MDX-10.

8. Use of an antibody and an IL-21 polypeptide for the manufacture of a medicament for treating cancer, wherein said antibody is selected from:
(i) HuMax-CD20, alemtuzumab, and Zamyl;
(ii) an antibody against tissue factor, laminin-5, EGF receptor, VEGF receptor, PDGF receptor, HER-2/neu, prostate-specific antigen (PSA), prostate stem cell antigen (PSCA), carcinoembryonic antigen (CEA), cancer antigen 125 (CA125), tumor-associated calcium signal transducer antigen (KSA), or VEGF;
(iii) an antibody that activates a TRAIL receptor; or
(iii) a monoclonal antibody against CTLA-4, leukocyte immunoglobulin-like receptor 1 (LIR), CD94, or NKG2A.

9. Use according to Claim 8, wherein the cancer is selected from the group consisting of metastatic malignant melanoma, renal cell carcinoma, ovarian cancer, small-cell lung cancer, non-small-cell lung cancer, breast cancer, colorectal cancer, prostate cancer, pancreatic cancer, bladder cancer, esophageal cancer, cervical cancer, endometrial cancer, lymphoma, and leukemia.

10. Use according to Claim 8 or 9, wherein the IL-21 polypeptide is to be administered prior to administration of the antibody.

11. Use according to Claim 8 or 9, wherein the IL-21 polypeptide is to be administered simultaneously with the antibody.

12. Use according to Claim 8 or 9, wherein the antibody is to be administered prior to administration of the IL-21 polypeptide.

13. Use according to any of Claims 8-12, wherein the antibody is HuMax-CD20.

14. Use according to any of Claims 8-12, wherein said anti-VEGF antibody is bevacizumab (avastin), wherein said anti-EGF-R antibody is HuMax-EGFr, wherein said anti-Her2/neu antibody is pertuzumab, or wherein said anti-CTLA-4 antibody is MDX-10.
